# EUROPEAN PATENT APPLICATION

(11) **EP 4 197 555 A1**
(43) Date of publication of application: **21.06.2023**
(21) Application number: 21857625.4
(22) Date of filing: 16.08.2021
(51) Int. Cl.: A61K 39/395, A61P 35/00, C07K 16/28, C07K 16/30

(54) **ANTI-CD73 ANTIBODY AND USE THEREOF**

(30) Priority: 17.08.2020 CN 202010828886; 23.10.2020 CN 202011152518
(71) Applicant: Akeso Biopharma, Inc., Zhongshan, Guangdong 528437 (CN)
(72) Inventor: WANG, Zhongmin, Zhongshan Guangdong 528437 (CN); JIN, Xiaoping, Zhongshan Guangdong 528437 (CN); LI, Baiyong, Zhongshan Guangdong 528437 (CN); XIA, Yu, Zhongshan Guangdong 528437 (CN)
(74) Representative: Cooley (UK) LLP
(86) International application number: PCT/CN2021/112771
(87) International publication number: WO 2022/037531

(57) **Abstract**

The present disclosure relates to an anti-CD73 antibody and application thereof in treating diseases caused by coronavirus infection. Specifically, a heavy chain variable region of the antibody contains amino acid sequences such as HCDR1 - HCDR3 as shown in SEQ ID NOs: 15-17; and the light chain variable region of the antibody contains amino acid sequences such as LCDR1 - LCDR3 as shown in SEQ ID NOs: 18 - 20.

## Description

### Technical Field

The present disclosure relates to the field of immunology. In particular, it relates to an anti-CD73 antibody and use thereof.

### Background Art

Ecto-5'-nucleotidase, i.e., an CD73 protein, is a multifunctional glycoprotein encoded by the NT5E gene with a protein molecular weight of 70 KD, and is anchored to the cell membrane by glyocsyl phosphatidy linositol (GPI) (Zimmermann H. Biochem J. 1992; 285: 345 - 365).

CD73 is widely distributed on the surface of human tissue cells and is also widely expressed on the surface of immune cells, such as dendritic cells, regulatory T-cells (Tregs), natural killer cells (NK cells), and myeloid-derived suppressor cells (MDSCs).

CD73 has both hydrolase and non-hydrolase activity. The immunosuppressive mechanism of CD73 enzymatic and non-enzymatic functions is mediated by the CD73-adenosine metabolic signaling pathway. CD39 upstream of CD73 is capable of catalyzing the production of adenosine monophosphate (AMP) by ATP, and the produced AMP is converted to adenosine by CD73. Adenosine binds downstream adenosine receptors (A2AR), which inhibit a series of immune activation-related signaling pathways such as LCK, MAPK, PKC and inhibits the immune killing of T-cells by activating protein kinase A (PKA) and Csk, thereby exerting immunosuppressive effects (Antonioli L, et al Nat Rev Cancer. 2013; 13: 842 - 857.)

Coronaviridae viruses include 2019 novel coronavirus (2019-nCoV or SARS-CoV-2, which induces novel coronavirus pneumonia COVID-19), HCoV-229E, HCoV-OC43, HCoV-NL63, HCoV-HKU1, SARS-CoV (which induces severe acute respiratory syndrome), and MERS-CoV (which induces Middle East respiratory syndrome). The above viral infections may cause severe clinical symptoms or may even be life-threatening.

SARS-CoV fights the immune response through different mechanisms. One of these mechanisms inhibits type 1 interferon (IFN-1) expression and signaling (Kindler, E., Thiel et al. SARS-CoV and IFN: Too Little, Too Late. Cell Host Microbe 2016, 19 (2), 139 - 141.). Inducible type I interferon plays a key role in the antiviral immune response by inducing cell resistance to viral infection and promoting apoptosis of virally infected cells. SARS-CoV hinders the rapid increase in IFN-I, which promotes the replication of coronavirus. ATP promotes IFN production, and it has been proven that improved extracellular ATP is able to reverse this cycle by promoting IFN secretions through the signaling pathway P38/JNK/ATF-2. Thus, cells lacking ATP are more susceptible to viral infections, such as SARS-CoV and COVID-19 (Taghizadeh-Hesary F., Akbari H. The Powerful Immune System Against Powerful COVID-19: A Hypothesis. Medical Hypotheses, 2020, 109762.). In vitro experiments have shown that SARS-CoV-2 expresses multiple proteins that inhibit interferon (Konno, Yoriyuki, et al. SARS-CoV-2 ORF3b is a potent interferon antagonist whose activity is further increased by a naturally occurring elongation variant. bioRxiv (2020 Yuen, Chun-Kit, et al. SARS-CoV-2 nsp13, nsp14, nsp15 and orf6 function as potent interferon antagonists. Emerging Microbes and Infections (2020): 1 - 29.), demonstrating that this is a key mechanism for inhibiting effective early immune responses.

In addition to its role in the acute phase, interferon also plays a critical role in the development of immunosuppressive Tregs. Lung injury in patients with severe COVID-19 is associated with cytokine release syndrome (CRS), indicating that immunosuppressive mechanisms may not be activated in time (Acharya, D., Liu, G. & Gack, M.U. Dysregulation of type I interferon responses in COVID-19. Nat Rev Immunol 20, 397 - 398 (2020). This is supported by the Treg count in patients with COVID-19, which is inversely proportional to disease severity.

Inhibition of the ATP degradation pathway may be used as a mechanism for treating COVID-19. ATP stimulates the secretion of interferon, which may fight the powerful interferon inhibition mechanism of SARS-CoV-2. If successful, this would help to eliminate the virus in the early stages of infection.

Two of the clinical manifestations of patients with COVID-19 are hypoxia and hypoxemia. Upregulation of hypoxia inducible factor-1 (HIF-1) and other molecules result in widespread expression of CD73 (Synnestvedt K, et al. J Clin Invest. 2002; 110: 993 - 1002.). In addition, A2B receptors are more easily activated in hypoxia. Activation of A2BR directly promotes the production of large amounts of IL-6 by relevant cells, which induces the differentiation of lung fibrocytes (Hongyan Zhong, Luiz Belardinelli et al. Synergy between A2B Adenosine Receptors and Hypoxia in Activating Human Lung Fibroblasts. Am J Respir Cell Mol Biol, 2005, 32: 2 - 8.). Moreover, IL-6 is one of the key inflammatory factors in cytokine storm in COVID-19 patients. Thus, reducing adenosine levels may be particularly beneficial for patients with CRS and respiratory distress. A number of studies have reported that IL-6 and GM-CSF are significantly elevated in patients with severe and critical COVID-19 (Characteristics of lymphocyte subsets and cytokines in peripheral blood of 123 hospitalized patients with 2019 novel coronavirus pneumonia (NCP). Wan SX, Yi QJ, Fan SB, Lv JL, Zhang XX, Guo L, Lang CH, Xiao Q, Xiao KH, et al. medRxiv 02/10/2020. 20021832; Chen Lei, Liu Huiguo, Liu Wei et al. Analysis of clinical characteristics of 29 patients with COVID-19. Chinese Journal of Tuberculosis and Respiratory Diseases, 2020, 43: E005). Studies on severe acute respiratory syndrome coronavirus (SARS-CoV) infection have also confirmed the mechanism by which SARS-CoV 3a protein directly activates NLRP3 inflammasomes in macrophages and mediates downstream IL-6 elevations (Chen IY, Moriyama M, Chang MF, Ichinohe T. Severe Respiratory Syndrome Coronavirus Viroporin 3a Activates the NLRP3 Inflammasome. Front Microbiol. eCollection 2019).

Enhancing the immune response may lead to faster viral clearance, shorter recovery times, fewer complications, longer immunity, and prevention of re-infection. The ability to enhance immune response presents a potential opportunity to treat COVID-19 and other epidemics.

CD73 is expressed in human B cells, T-cells, myelocytes, bone marrow stromal cells and thymic epithelial cells. Studies have shown that (Giovanni Forte, Rosalinda Sorrentino et al. Inhibition of CD73 Improves B Cell-Mediated Anti-Tumor Immunity in a Mouse Model of Melanoma. The Journal of Immunology, 2012, 189: 2226 - 2233.) antagonism of CD73 may affect the activity of B cells through IL-17A in the production of IgG, which is a major component of anti-bacterial, antitoxin and antiviral antibodies. It was found in the autopsy of patients who died of COVID-19 caused by infection of the same Coronaviridae virus that the number of CD4+ and CD8+ T-cells significantly decreased in the blood of the patients, but the proportion of Th17 cells producing IL-17A was significantly higher (Xu Z, Shi L, Wang YJ, Zhang JY, Huang L, Zhang C et al. Pathological findings of COVID-19 associated with acute respiratory distress syndrome. The Lancet Respiratory Medicine. 2020).

In summary, blocking CD73 may be used to treat diseases induced by Coronaviridae viruses, including COVID-19, by regulating the CD73 adenosine pathway and affecting the immune response.

### Summary of the Invention

The inventors of the present disclosure use a mammalian cell expression system that expresses recombinant human CD73 as an antigen to immunize mice, and obtain hybridomas by fusing mouse splenocytes with myeloma cells. The inventors obtained the hybridoma cell line LT014 (preservation number CCTCC NO: C2018137) by screening a large number of samples.

The inventors of the present disclosure surprisingly found that hybridoma cell line LT014 was capable of secreting and producing a specific monoclonal antibody (named 19F3) that specifically bound to human CD73, respectively. Further, the inventors of the present disclosure prepared an anti-human CD73 humanized antibody (named 19F3H2L3).

The inventors of the present disclosure also surprisingly found that the antibody 19F3H2L3 of the present disclosure was highly effective in inhibiting the enzyme activity of CD73 in a non-substrate competitive manner, reducing the production of adenosine, promoting endocytosis of CD73 on the cell membrane surface, and stimulating the activation and proliferation of B cells. The antibody of the present disclosure is capable of treating diseases associated with coronavirus infection.

One aspect of the present disclosure relates to an anti-CD73 (e.g., human CD73) antibody or an antigen-binding fragment thereof for treating and/or preventing infections caused by coronavirus, in which the anti-CD73 antibody contains:
or the anti-CD73 antibody contains HCDR1, HCDR2 and HCDR3 contained in the heavy chain variable region as shown in SEQ ID NO: 2, SEQ ID NO: 6 or SEQ ID NO: 10, and LCDR1, LCDR2 and LCDR3 contained in the light chain variable region as shown in SEQ ID NO: 4, SEQ ID NO: 8, SEQ ID NO: 12 or SEQ ID NO: 14;
preferably, according to the IMGT numbering system, the anti-CD73 antibody contains: HCDR1, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 15, or an amino acid sequence having one or a plurality of (preferably 1, 2, or 3) conservative amino acid mutations (preferably substitutions, insertions, or deletions) as compared to the sequence, or consists thereof;
HCDR2, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 16, or an amino acid sequence having one or a plurality of (preferably 1, 2, or 3) conservative amino acid mutations (preferably substitutions, insertions, or deletions) as compared to the sequence, or consists thereof;
HCDR3, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 17, or an amino acid sequence having one or a plurality of (preferably 1, 2, or 3) conservative amino acid mutations (preferably substitutions, insertions, or deletions) as compared to the sequence, or consists thereof;
LCDR1, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 18, or an amino acid sequence having one or a plurality of (preferably 1, 2, or 3) conservative amino acid mutations (preferably substitutions, insertions, or deletions) as compared to the sequence, or consists thereof;
LCDR2, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 19, or an amino acid sequence having one or a plurality of (preferably 1, 2, or 3) conservative amino acid mutations (preferably substitutions, insertions, or deletions) as compared to the sequence, or consists thereof; and
LCDR3, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 20, or an amino acid sequence having one or a plurality of (preferably 1, 2, or 3) conservative amino acid mutations (preferably substitutions, insertions, or deletions) as compared to the sequence, or consists thereof.

In some embodiments of the present disclosure,
the heavy chain variable region of the antibody contains the following sequence, or consists thereof:
   SEQ ID NO: 2, SEQ ID NO: 6, or SEQ ID NO: 10, a sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity thereto, or an amino acid sequence having one or a plurality of (preferably 1, 2, 3, 4, 5, 6, 7, 8, 9, or 10) conservative amino acid mutations (preferably substitutions, insertions, or deletions) as compared to the amino acid sequence as shown in SEQ ID NO: 2, SEQ ID NO: 6 or SEQ ID NO: 10; and
the V_{L} of the antibody contains the following sequence, or consists thereof:
   SEQ ID NO: 4, SEQ ID NO: 8, SEQ ID NO: 12 or SEQ ID NO: 14, a sequence having at least 80%, 81%, 82%, 83%, 84%, 85%, 86%, 87%, 88%, 89%, 90%, 91%, 92%, 93%, 94%, 95%, 96%, 97%, 98%, or 99% sequence identity thereto, or an amino acid sequence having one or a plurality of (preferably 1, 2, 3, 4, 5, 6, 7, 8, 9, or 10) conservative amino acid mutations (preferably substitutions, insertions, or deletions) as compared to the amino acid sequence as shown in SEQ ID NO: 4, SEQ ID NO: 8, SEQ ID NO: 12 or SEQ ID NO: 14.

In some embodiments of the present disclosure, the amino acid sequence of the heavy chain variable region of the antibody is as shown in SEQ ID NO: 2, and the amino acid sequence of the light chain variable region of the antibody is as shown in SEQ ID NO: 4;
the amino acid sequence of the heavy chain variable region of the antibody is as shown in SEQ ID NO: 6, and the amino acid sequence of the light chain variable region of the antibody is as shown in SEQ ID NO: 8;
the amino acid sequence of the heavy chain variable region of the antibody is as shown in SEQ ID NO: 10, and the amino acid sequence of the light chain variable region of the antibody is as shown in SEQ ID NO: 12; or
the amino acid sequence of the heavy chain variable region of the antibody is as shown in SEQ ID NO: 10, and the amino acid sequence of the light chain variable region of the antibody is as shown in SEQ ID NO: 14.

In some embodiments of the present disclosure, the heavy chain constant region of the antibody is the Ig gamma-1 chain C region, ACCESSION: P01857; the light chain constant region is the Ig kappa chain C region, ACCESSION: P01834.

The variable regions of the light chain and heavy chain determine antigen binding; the variable regions of each chain contain three hypervariable regions called complementarity determining regions (CDRs) (CDRs of the heavy chain (H) contain HCDR1, HCDR2, and HCDR3, and CDRs of the light chain (L) contain LCDR1, LCDR2, LCDR3; which are named by Kabat et al., see Bethesda M.d., Sequences of Proteins of Immunological Interest, Fifth Edition, NIH Publication 1991; 1 - 3: 91 - 3242.

Preferably, CDRs may also be defined by the IMGT numbering system, please refer to Ehrenmann, Francois, Quentin Kaas, and Marie-Paule Lefranc. IMGT/3Dstructure-DB and IMGT/DomainGapAlign: a database and a tool for immunoglobulins or antibodies, T cell receptors, MHC, IgSF and MhcSF. Nucleic acids research 2009; 38(suppl_1): D301 - D307.

The amino acid sequences of the CDRs of monoclonal antibody sequences are analyzed according to IMGT definitions by means of techniques well known to those skilled in the art, for example, by means of a VBASE2 database.

Where antibody heavy chain and light chain variable region sequences are known, there are several methods for determining antibody CDRs, including Kabat, IMGT, Chothia, and AbM numbering systems.

However, the application of each CDR definition with respect to an antibody or variant thereof are within the scope of the terms defined and used herein. If the variable region amino acid sequence of the antibody is given, those skilled in the art are generally capable of determining which residues contain a particular CDR without relying on any experimental data other than the sequence itself.

The antibodies 19F3, 19F3H1L1, 19F3H2L2, and 19F3H2L3 to which the present disclosure relates have the same CDR:
the amino acid sequences of the three CDRs of the heavy chain variable region thereof are as follows:
   HCDR1: GYSFTGYT (SEQ ID NO: 15),
   HCDR2: INPYNAGT (SEQ ID NO: 16),
   HCDR3: ARSEYRYGGDYFDY (SEQ ID NO: 17);
the amino acid sequences of the 3 CDRs of the light chain variable region thereof are as follows:
   LCDR1: QSLLNSSNQKNY (SEQ ID NO: 18),
   LCDR2: FAS (SEQ ID NO: 19),
   LCDR3: QQHYDTPYT (SEQ ID NO: 20).

In some embodiments of the present disclosure, the antibody is a monoclonal antibody. In some embodiments of the present disclosure, the antibody is a humanized antibody, a chimeric antibody, or a multispecific antibody (e.g., a bispecific antibody), preferably, the heavy chain amino acid sequence of the anti-CD73 antibody is as shown in SEQ ID NO: 23 and the light chain amino acid sequence is as shown in SEQ ID NO: 24.

In some examples of the present disclosure, the antigen-binding fragment is selected from a Fab, a Fab', a F(ab')₂, a Fd, a Fv, a dAb, a Fab/c, a CDR fragment, a single chain antibody (e.g., scFv), a humanized antibody, a chimeric antibody, or a bispecific antibody.

Another aspect of the present disclosure relates to a conjugate for treating and/or preventing infections caused by coronavirus, comprising an antibody and a coupling portion, in which the antibody is an anti-CD73 antibody or antigen-binding fragment thereof in any item of the present disclosure, the coupling portion is a purification tag (such as a His tag), a detectable label or a small molecule drug; preferably, the coupling portion is a radionuclide, a fluorescent substance, a chemiluminescent substance, a colored substance, polyethylene glycol or an enzyme; preferably, the small molecule drug is a small molecule cytotoxic drug; more preferably, the small molecule drug is a tumor chemotherapy drug, more preferably, the antibody or antigen-binding fragment thereof is linked to the small molecule drug by a linker; for example, the linker is a hydrazone bond, a disulfide bond, or a peptide bond; more preferably, the antibody or antigen-binding fragment thereof is linked to the small molecule drug at a certain molar ratio; for example, the molar ratio is 1: (2 - 4).

Yet another aspect of the present disclosure relates to a fusion protein or a multispecific antibody (preferably a bispecific antibody) for treating and preventing infections caused by coronavirus, the fusion protein or multispecific antibody containing the anti-CD73 antibody or antigen-binding fragment thereof of any item of the present disclosure.

Yet another aspect of the present disclosure relates to a kit for treating and preventing infections caused by coronavirus, the kit containing an effective dose (e.g., 0.001 mg - 1,000 mg) of the anti-CD73 antibody or antigen-binding fragment thereof of the present disclosure, a conjugate, a fusion protein or a multispecific antibody of the present disclosure, and optionally, further containing an effective dose of one or a plurality of antiviral drugs (e.g., 100 - 2,400 mg).

Yet another aspect of the present disclosure relates to a kit for treating and preventing infections caused by coronavirus, the kit comprising the anti-CD73 antibody or antigen-binding fragment thereof of the present disclosure, a conjugate, a fusion protein or a multispecific antibody of the present disclosure, and an antigen used as a vaccine that is selected from virus, bacteria, fungi, rickettsia, chlamydia, mycoplasma, parasite, prion or tumor.

Yet another aspect of the present disclosure relates to a pharmaceutical composition for treating and preventing infections caused by coronavirus, the pharmaceutical composition containing the anti-CD73 antibody or antigen-binding fragment thereof of any item of the present disclosure, and a conjugate, a fusion protein, or a multispecific antibody of the present disclosure; optionally, the pharmaceutical composition further comprises a pharmaceutically acceptable vector and/or excipient. Preferably, the pharmaceutical composition is in a form suitable for administration by subcutaneous injection, intradermal injection, intravenous injection, intramuscular injection or intralesional injection.

Yet another aspect of the present disclosure relates to the purpose of use of the antibody or antigen-binding fragment thereof, the conjugate, or the fusion protein or multispecific antibody for treating and preventing infections caused by coronavirus in the preparation of a drug or kit for treating and/or preventing a coronavirus infection, which is preferably selected from novel coronaviruses SARS-CoV-2, HCoV-229E, HCoV-OC43, HCoV-NL63, HCoV-HKU1, SARS-CoV, and/or MERS-CoV.

Yet another aspect of the present disclosure relates to a method for treating and/or preventing a coronavirus infection, comprising administering an effective dose of the anti-CD73 antibody or antigen-binding fragment thereof, the conjugate, or the fusion protein or multispecific antibody to a subject or patient, preferably, the Coronaviridae virus is selected from novel coronaviruses SARS-CoV-2, HCoV-229E, HCoV-OC43, HCoV-NL63, HCoV-HKU1, SARS-CoV, and/or MERS-CoV. In some embodiments, the method comprises concurrently or sequentially administering an antiviral drug (such as favipiravir) to a subject or patient, preferably, the antiviral drug is an RNA virus inhibitor.

In some embodiments, the effective dose of the anti-CD73 antibody or antigen-binding fragment thereof, the antibody-drug conjugate or the bispecific antibody of the present disclosure is 0.001 mg - 1,000 mg, more preferably 0.001 mg - 900 mg, 0.001 mg - 800 mg, 0.001 mg - 700 mg, 0.001 mg - 600 mg, 0.001 mg - 500 mg, 0.001 mg - 400 mg, 0.001 mg - 300 mg, 0.001 mg - 200 mg, or 0.001 mg - 100 mg, most preferably 100 mg, 200 mg, 300 mg, 400 mg, 500 mg, 600 mg, 700 mg, 800 mg, 900 mg or 1,000 mg, or the effective dose is 0.1 - 100 mg/kg, preferably 1 - 90 mg/kg, 1 - 80 mg/kg, 1-70 mg/kg, 1 - 60 mg/kg, 1-50 mg/kg, 1 - 40 mg/kg, 1-30 mg/kg, 1 - 20 mg/kg, or 1 - 10 mg/kg based on the weight of the subject or patient.
In any of the above embodiments of the present disclosure, the effective dose of one or a plurality of antiviral drugs (e.g., favipiravir) is 100 - 2,400 mg, preferably 100 mg - 2,300 mg, 100 mg - 2,200 mg, 100 mg - 2,100 mg, 100 mg - 2,000 mg, 100 mg - 1,900 mg, 100 mg - 1,800 mg, 100 mg - 1,700 mg, 100 mg - 1,600 mg, 100 mg - 1,800 mg, 100 mg - 1,800 mg, 100 mg - 1,800 mg, 100 mg - 1,800 mg, or 100 mg - 1,800 mg, more preferably 100 mg, 200 mg, 300 mg, 400 mg, 500 mg, 600 mg, 700 mg, 800 mg, 900 mg, or 1,000 mg. Alternatively, in any of the above embodiments of the present disclosure, the effective dose of the antiviral drug is 0.1 - 100 mg/kg, preferably 1 - 90 mg/kg, 1 - 80 mg/kg, 1 - 70 mg/kg, 1 - 60 mg/kg, 1 - 50 mg/kg, 1 - 40 mg/kg, 1 - 30 mg/kg, 1 - 20 mg/kg, or 1 - 10 mg/kg, based on the weight of the subject or patient. Another aspect of the present disclosure relates to a single drug dosage unit containing 0.001 mg - 1,000 mg of the antibody or antigen-binding fragment thereof of the present disclosure, preferably 0.001 mg - 900 mg, 0.001 mg - 800 mg, 0.001 mg - 700 mg, 0.001 mg - 600 mg, 0.001 mg - 500 mg, 0.001 mg - 400 mg, 0.001 mg - 300 mg, 0.001 mg - 200 mg, or 0.001 mg - 100 mg, more preferably 100 mg, 200 mg, 300 mg, 400 mg, 500 mg, 600 mg, 700 mg, 800 mg, 900 mg, or 1,000 mg of the antibody or antigen-binding fragment thereof of the present disclosure.

Yet another aspect of the present disclosure relates to hybridoma cell line LT014, which was preserved at the China Center for Type Culture Collection (CCTCC) in Wuhan, China on June 21, 2018, with the preservation number CCTCC NO: C2018137.

Another aspect of the present disclosure also relates to the anti-CD73 antibody or antigen-binding fragment, conjugate, or fusion protein or multispecific antibody of the present disclosure for use as an auxiliary to enhance the immune response of an organism to an antigen. In some embodiments, the antigen is derived from a virus, bacteria, fungi, rickettsia, chlamydia, mycoplasma, parasite, prion, or tumor;
preferably, the virus includes an RNA virus and a DNA virus;
preferably, the RNA virus includes a Coronaviridae virus;
preferably, the coronavirus includes the 2019 novel coronavirus (2019-nCoV or SARS-CoV-2, which induces COVID-19), HCoV-229E, HCoV-OC43, HCoV-NL63, HCoV-HKU1, SARS-CoV (which induces severe acute respiratory syndrome), and MERS-CoV (which induces Middle East respiratory syndrome).

Another aspect of the invention relates to a method of increasing vaccine efficacy or enhancing an organism's responsiveness to a vaccine, comprising administering the anti-CD73 antibody or antigen-binding fragment, conjugate, fusion protein or multispecific antibody of the present disclosure to the subject during, before, or after vaccination, preferably the antigen contained in the vaccine is derived from a virus, bacteria, fungi, rickettsia, chlamydia, mycoplasma, parasite, prion or tumor;
preferably, the virus includes an RNA virus and a DNA virus;
preferably, the RNA virus includes a coronavirus;
preferably, the coronavirus includes the 2019 novel coronavirus (2019-nCoV or SARS-CoV-2, which induces COVID-19), HCoV-229E, HCoV-OC43, HCoV-NL63, HCoV-HKU1, SARS-CoV (which induces severe acute respiratory syndrome), and MERS-CoV (which induces Middle East respiratory syndrome).

In some embodiments, the anti-CD73 antibody or antigen-binding fragment thereof, conjugate, fusion protein, or multispecific antibody is administered (preferably intravenously) one or a plurality of times.

In the present disclosure, scientific and technical terms used herein have the meanings commonly understood by those skilled in the art, unless otherwise specified. Moreover, the cell culture, molecular genetics, nucleic acid chemistry, and immunology laboratory operating procedures used herein are all routine steps widely used in the respective arts. At the same time, for a better understanding of the present disclosure, definitions and explanations of relevant terms are provided below.

As used herein, the term EC₅₀ refers to concentration for 50% of maximal effect, which refers to a concentration that is capable of inducing 50% of the maximal effect.

As used herein, the term "antibody" refers to an immunoglobulin molecule that typically consists of two pairs of polypeptide chains (each pair having a "light" (L) chain and a "heavy" (H) chain). Antibody light chains may be classified as κ and λ light chains. Heavy chains may be classified as µ, δ, γ, α, or ε, and isotypes of antibodies are defined as IgM, IgD, IgG, IgA, and IgE, respectively. Within the light chain and heavy chain, the variable region and the constant region are linked by a "J" region of approximately 12 or more amino acids, the heavy chain further containing a "D" region of approximately 3 or more amino acids. Each heavy chain consists of a heavy chain variable region (V_{H}) and a heavy chain constant region (C_{H}). The C_{H} consists of 3 domains (C_{H1}, C_{H2}, and C_{H3}). Each light chain consists of a light chain variable region (V_{L) and a light chain constant region (C}L). The C_{L} consists of one domain C_{L}. The constant region of the antibody is capable of mediating the binding of immunoglobulins to host tissues or factors, including various cells of the immune system (e.g., effector cells) and the first component (C1q) of the classical complement system. The V_{H} and V_{L} regions may also be subdivided into hypervariable regions (called complementarity-determining regions (CDRs) interspersed with more conservative regions called framework regions (FRs). Each V_{H} and V_{L} is in the following order: FR1, CDR1, FR2, CDR2, FR3, CDR3, and FR4, consisting of 3 CDRs and 4 FRs arranged from amino-terminus to carboxy-terminus. The variable regions (V_{H} and V_{L}) of each heavy chain/light chain pair form an antigen binding site, respectively. The allocation of amino acids to each region or domain follows Kabat Sequences of Proteins of Immunological Interest (National Institutes of Health, Bethesda M.d. (1987 and 1991), or Chothia & Lesk J. Mol. Biol. 1987; 196: 901 - 917; Chothia et al. Nature 1989; 342: 878 - 883 or IMGT numbering system as defined in Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 2009; 38(suppl_1): D301 - D307. The term "antibody" is not limited by any particular antibody production method. For example, it includes, in particular, recombinant antibodies, monoclonal antibodies, and polyclonal antibodies. Antibodies may be antibodies of different isotypes, for example, IgG (e.g., IgG1, IgG2, IgG3, or IgG4 subtype), IgA1, IgA2, IgD, IgE, or IgM antibodies.

As used herein, the terms "mAb" and "monoclonal antibody" refer to an antibody or fragment of an antibody from a group of highly homologous antibody molecules, and also refer to a group of antibody molecules that are identical except for natural mutations that may occur spontaneously. mAbs are highly specific for a single epitope on an antigen. Polyclonal antibodies, relative to monoclonal antibodies, typically comprise at least 2 or more different antibodies that typically recognize different epitopes on an antigen. Monoclonal antibodies are typically obtained using the hybridoma technology first reported by Kohler et al. (Köhler G, Milstein C. Continuous cultures of fused cells secreting antibody of predefined specificity [J]. nature, 1975; 256 (5517): 495), but may also be obtained using recombinant DNA technology (refer to U.S. Patent 4, 816, 567).

As used herein, the term "humanized antibody" refers to an antibody or antibody fragment obtained after all or part of a CDR of a human immunoglobulin (receptor antibody) is replaced with a CDR of a non-human antibody (donor antibody), in which the donor antibody may be a non-human (e.g., mouse, rat, or rabbit) antibody with an expected specificity, affinity, or reactivity. In addition, some amino acid residues of the FR of the receptor antibody may also be replaced with amino acid residues of the corresponding non-human antibody, or with amino acid residues of other antibodies, in order to further refine or optimize the performance of the antibody. For more details on humanized antibodies, refer to, for example, Jones et al., Nature 1986; 321: 522 - 525; Reichmann et al., Nature 1988; 332: 323 - 329; Presta, Curr. Op. Struct. Biol., 1992; 2: 593 - 596; and Clark M. Antibody humanization: a case of the 'Emperor's new clothes'?[J]. Immunol. Today, 2000; 21 (8): 397 - 402.

As used herein, the term "separated" or "isolated" refers to obtained manually in a natural state. If an "separate" substance or ingredient occurs in nature, the natural environment it is in may have changed, or the substance is isolated from the natural environment, or both. For example, a certain unisolated polynucleotide or polypeptide is naturally present in a living animal, and a high purity identical polynucleotide or polypeptide separated from such a natural state is referred to as separated. The term "separated" or "isolated" does not exclude substances mixed with man-made or synthetic material, nor does it exclude the presence of other impure substances that do not affect the activity of the substance.

As used herein, the term "vector" refers to a nucleic acid vehicle into which a polynucleotide may be inserted. A vector that enables expression of an inserted polynucleotide-encoded protein is referred to as an expression vector. A vector may be introduced into the host cell by transformation, transduction or transfection, so that the genetic material element carried thereby is expressed in the host cell. Vectors are well known by those skilled in the art, including but not limited to: plasmids; bacteriophages; cosmids; artificial chromosomes, for example, yeast artificial chromosomes (YACs), bacterial artificial chromosomes (BACs), or P1-derived artificial chromosomes (PACs); examples of bacteriophages are λ bacteriophages or M13 bacteriophages and animal viruses, etc. Animal viruses that may be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviricetes (such as SV40). A vector may contain a variety of elements that control expression, including, but not limited to, promoter sequences, transcriptional start sequences, enhancer sequences, selection elements, and reporter genes. In addition, the vector may also contain an origin of replication.

As used herein, the term "host cells" refers to cells that may be used to introduce a vector, including, but not limited to, prokaryotic cell such as Escherichia coli or Bacillus subtilis, fungal cells such as yeast cells or aspergillus, insect cells such as S2 Dropsophila cells or Sf9, or animal cells such as fibroblasts, CHO cells, COS cells, NSO cells, HeLa cells, GS cells, BHK cells, HEK 293 cells, or human cells.

As used herein, the term "specific binding" refers to a non-random binding reaction between two molecules, such as a reaction between an antibody and the antigen against which it is directed. In certain embodiments, an antibody that specifically binds to an antigen (or an antibody that is specific to an antigen) refers to an antibody that binds to the antigen with an affinity (K_{D}) of less than approximately 10⁻⁵ M, for example, less than approximately 10⁻⁶ M, 10⁻⁷ M, 10⁻⁸ M, 10⁻⁹ M, 10⁻¹⁰ M or less.

As used herein, the term "K_{D}" refers to a dissociation equilibrium constant for a particular antibody-antigen interaction that is used to describe binding affinity between an antibody and an antigen. The smaller the dissociation equilibrium constant, the tighter the antibody-antigen binding, and the higher the affinity between the antibody and the antigen. Typically, the antibody binds to an antigen (e.g., a PD-1 protein) with a dissociation equilibrium constant (K_{D}) of less than approximately 10⁻⁵ M, for example, less than approximately 10⁻⁶ M, 10⁻⁷ M, 10⁻⁸ M, 10⁻⁹ M, 10⁻¹⁰ M or less. K_{D} may be determined using methods familiar to those skilled in the art, for example, using Fortebio molecular interactions instrument.

As used herein, the terms "mAb" and "monoclonal antibody" have the same meaning and may be used interchangeably; the terms "polyclonal antibody" and "pAb" have the same meaning and may be used interchangeably; the terms "polypeptide" and "protein" have the same meaning and may be used interchangeably. Also, in the present disclosure, amino acids are typically represented by single- and three-letter abbreviations that are well known in the art. For example, alanine may be represented by A or Ala.

As used herein, the term "pharmaceutically acceptable vector and/or excipient" refers to a vector and/or excipient that is well known in the art to be pharmacologically and/or physiologically compatible with the subject and the active ingredient (refer to, e.g., Remington's Pharmaceutical Sciences. Edited by Gennaro AR, 19th ed. Pennsylvania: Mack Publishing Company, 1995) and includes, but is not limited to: pH modifiers, surfactants, adjuvants, and ionic strength enhancers. For example, pH modifiers include, but are not limited to, phosphate-buffered saline; surfactants include, but are not limited to, cationic, anionic, or nonionic surfactants, such as Tween-80; ionic strength enhancers include, but are not limited to, sodium chloride.

In the present disclosure, the term "single drug dosage unit" denotes a single drug dosage form of an antibody or antigen-binding fragment thereof, the antibody-drug conjugate, or the bispecific antibody (or pharmaceutical composition containing thereof) of the present disclosure to be administered to a subject or patient at the time points in the dosing regimen, such as in units of one ampoule. As used herein, the term "effective dose" refers to a dose sufficient to obtain or at least partially obtain a desired effect. For example, an effective dose to prevent a disease (e.g., a disease caused by a Coronaviridae virus, preferably COVID-19) refers to a dose sufficient to prevent, stop, or delay the occurrence of a disease; an effective dose to treat a disease refers to a dose sufficient to cure or at least partially stop the disease and the complications thereof in a patient who already has a disease.

### Beneficial Effects of the Invention:

The monoclonal antibodies of the present disclosure are capable of binding to CD73 with good specificity, and are capable of very effectively inhibiting the enzyme activity of CD73 in a non-substrate-competitive manner and inducing endocytosis of CD73 on the cell surface, thereby reducing the production of adenosine; they are also capable of stimulating the activation and proliferation of B cells in an adenosine-independent manner.

### Description of Attached Drawings

Fig. 1. MFI Value of 19F3H2L3-induced B-cell CD69.
Fig. 2. MFI Value of 19F3H2L3-induced B-cell CD83.
Fig. 3. Enzyme Activity Detection Result of MDA-MB-231 Cells Added with Anti-CD73 Antibody.
Fig. 4. Enzyme Activity Detection Result of U87-MG Cells Added with Anti-CD73 Antibody.
Fig. 5. Detection Result after Addition of Anti-CD73 Antibody to MDA-MB-231 Cells to Induce CD73 Endocytosis on Cell Membrane Surface, in which Fig. 5A represents the MFI value and Fig. 5B shows the internalization rate.
Fig. 6. Detection Result after Addition of Anti-CD73 Antibody to U87-MG Cells to Induce CD73 Endocytosis on Cell Membrane Surface, in which Fig. 6A represents the MFI value and Fig. 6B is the internalization rate.
Fig. 7. Detection Result of Effectiveness of Anti-CD73 Antibody in Inducing Production of Antibodies against COVID-19 S Protein.
Fig. 8. Detection Result of Binding Activity of Anti-CD73 Antibody 19F3H2L3(hG1DM) to CD8+ T Cells and CD19+ B Cells as Detected by FACS.
Fig. 9. Detection Result of Inhibition of Enzyme Activity of CD73 Expressed by Human PBMC Membrane by Anti-CD73 Antibody 19F3H2L3(hG1DM).
Fig.10. Detection Result of Effective Induction of B Cell Activity by Anti-CD73 Antibody 19F3H2L3(hG1DM) as Detected by FACS.
Fig. 11. Detection Result of Stimulation of B Cell Proliferation by Anti-CD73 Antibody 19F3H2L3 (hG 1DM).

### Specific Embodiments

Examples of the present disclosure will be described in detail below in connection with embodiments. Those skilled in the art will appreciate that the following examples are for illustrative purposes only and are not to be considered as limiting the scope of the present disclosure. Where no specific technique or condition is noted in the examples, they are implemented according to the techniques or conditions described in the literature in the art (e.g., refer to Molecular Cloning: A Laboratory Manual by J. Sambrook et al. and translated by Huang Peitang et al., 3rd Edition, Science Press) or according to the product instructions. Conventional products of reagents or instruments which manufacturer is not specified may be purchased through the market. For example, MDA-MB-231 cells and U87-MG cells may be purchased from ATCC.

In the following examples of the present disclosure, BALB/c mice used were purchased from Guangdong Medical Laboratory Animal Center.

In the following examples of the present disclosure, the positive control antibody MEDI9447 (Oleclumab) used was manufactured by Akeso Biopharma Co., Ltd., the sequence thereof was constructed and synthesized with the Oleclumab amino acid sequence disclosed by Medlmmune Limited in the WHO drug information database and related patents (https://www.who.int/medicines/publications/druginformation/innlists/en/), and is labeled as MEDI9447 or MEDI9447 (Akeso) or MEDI-9447 (Akeso) in the examples;
in the following examples of the present disclosure, the control antibody CPI-006 used, with Mupadolimab as the generic name of the drug product, was manufactured by Akeso Biopharma Co., Ltd., the sequence thereof is consistent with the sequence in WHO, WHO Drug Information, Proposed INN: List 125. 2021, 35(2): 484 - 485. The heavy chain amino acid sequence of CPI-006 (Mupadolimab) is as shown in SEQ ID NO: 21, and the light chain amino acid sequence of CPI-006 is as shown in SEQ ID NO: 22.

In the following examples of the present disclosure, the sequence of the isotype control antibody, human anti-Hen Egg Lysozyme IgG (i.e., anti-HEL antibody, or human IgG, abbreviated as hIgG, or isotype control) used was from the variable region sequence of the Fab F10.6.6 sequence in the Affinity maturation increase the stability and plasticity of the Fv domain of anti-protein antibodies published by Acierno et al. (Acierno et al. J Mol Biol. 2007; 374 (1): 130 - 146.); the hIgGIDM used in the examples was an anti-HEL isotype control antibody with a hGIDM constant region sequence, and it was prepared in the laboratory of Akeso Biopharma Co., Ltd.

In the following examples of the present disclosure, the normal human peripheral blood used was from Zhongshan Blood Bank, and the human peripheral blood cells were isolated and prepared by Akeso Biopharma Co., Ltd., with informed consent obtained from the providers.

In the following examples of the present disclosure, the FITC-labeled anti-human CD3 antibody used was from Biolegend, catalog number: 344804; the PE-labeled anti-human CD19 antibody used was obtained from Biolegend, catalog number: 302254; the APC-labeled anti-human CD69 antibody used was from Biolegend, catalog number: 310910; the APC-labeled anti-human CD83 antibody used was from Biolegend, catalog number: 305312; the acridine orange staining solution used was from Thermofisher, catalog number: 93001; the propidium iodide staining solution used was from BD, catalog number: 51-66211E; the APC-labeled mouse IgG1 isotype control antibody used was from Biolegend, catalog number: 400122; the mouse IgG1 isotype control antibody used was from Thermofisher, catalog number: 10400C; the bovine serum albumin (BSA) used was from Sigma, catalog number: V900933-1KG; the RPMI 1640 used was from Gibco, catalog number: 22400-089; the fetal bovine serum used was from Excell bio, catalog number: FSP500; the sodium pyruvate used was from Gibco, catalog number: 11360-070; the non-essential amino acid used was from Gibco, catalog number: 11140-050; the L-glutamine used was from Gibco, catalog number: 25030-081; the MDA-MB-231 used was from ATCC, catalog number: HTB-26; the U87-MG used was from ATCC, catalog number: HTB-14; the CTG chromogen solution used was CellTiter-Glo^{®} One Solution Assay Kit from promega, catalog number: G8461; the Alexa Fluor^{®} 647-labeled mouse anti-human IgG secondary antibody used was from Southern Biotech, catalog number: 9040-31.

### Example 1: Preparation of anti-CD73 antibody 19F3

### 1. Preparation of hybridoma cell line LT014

The antigen used to prepare the anti-CD73 antibody was human NT5E-His (NT5E is GenbankID: NP_002517.1, position: 1-552). The splenocytes of immunized mice were fused with mouse myeloma cells to prepare hybridomas. With human NT5E (NT5E is GenbankID: NP_002517.1, position: 1-552)-Biotin as an antigen, hybridomas were screened by indirect ELISA to obtain hybridomas capable of secreting antibodies that specifically bind to CD73. A stable hybridoma line was obtained by performing dilution cloning on the screened hybridomas. The above hybridoma line is named hybridoma cell line LT014, respectively, and the secreted monoclonal antibody is named 19F3, respectively.

Hybridoma line LT014 (also known as CD73-19F3) was preserved at CCTCC on June 21, 2018, with the preservation number CCTCC NO: C2018137, and the preservation address is Wuhan, China. Wuhan University, postal code: 430072.

### 2. Preparation of anti-CD73 antibody 19F3

The LT014 cell line prepared above was cultured with CD medium (chemical defined medium) (CD medium containing 1% penicillin, cultured in a cell incubator at 5% CO₂, 37°C), respectively. After 7 days, the cell culture supernatant was harvested, centrifuged at high speed, vacuum filtered through a microporous membrane, and purified using HiTrap protein AHP column to prepare antibody 19F3, respectively.

### Example 2: Sequence analysis of anti-CD73 antibody 19F3

mRNA was extracted from the LT014 cell line cultured in Example 1 according to the method of the total RNA extraction kit for cultured cell/bacteria (Tiangen, catalog number DP430).

cDNA was synthesized according to the instructions of the Invitrogen SuperScript^{®} III First-Strand Synthesis System for RT-PCR kit and PCR amplification was performed.

TA cloning was directly performed on PCR amplification products. Refer to the instructions of the pEASY-T1 Cloning Kit (Transgen CT101) for the specific procedures.

The TA cloning product was directly sequenced, and the sequencing results are as follows:
The nucleic acid sequence of the V_{H} is as shown in SEQ ID NO: 1, and was 363 bp in length.

The amino acid sequence encoded thereby is as shown in SEQ ID NO: 2, and was 121aa in length;
in which the sequence of heavy chain CDR1 is as shown in SEQ ID NO: 15, the sequence of heavy chain CDR2 is as shown in SEQ ID NO: 16, and the sequence of heavy chain CDR3 is as shown in SEQ ID NO: 17.

The nucleic acid sequence of the V_{L} is as shown in SEQ ID NO: 3, and was 339 bp in length.

The amino acid sequence encoded thereby is as shown in SEQ ID NO: 4, and was 113aa in length;
in which the sequence of light chain CDR1 is as shown in SEQ ID NO: 18, the sequence of light chain CDR2 is as shown in SEQ ID NO: 19, and the sequence of light chain CDR3 is as shown in SEQ ID NO: 20.

### Example 3: Design and preparation of light chain and heavy chain of humanized antibodies to human CD73

### 1. Design of light chain and heavy chain of humanized antibodies 19F3H1L1, 19F3H2L2, and 19F3H2L3 to human CD73

The variable region sequence of antibodies 19F3H1L1, 19F3H2L2, 19F3H2L3 was obtained by mutations designed by a computer-simulated model according to the three-dimensional crystal structure of human CD73 protein (Hage T, Reinemer P, Sebald W. Crystals of a 1:1 complex between human interleukin-4 and the extracellular domain of its receptor alpha chain. Eur J Biochem. 1998; 258 (2): 831 - 6.) and the sequence of the antibody 19F3 obtained in Example 2 (antibody constant region sequence, from the database of NCBI, Ig gamma-1 chain C region, ACCESSION: P01857 was used for C_{H}; Ig kappa chain C region, ACCESSION: P01834 was used for C_{L}).

The designed variable region sequences are as follows:
(1) V_{H} and V_{L} sequences of humanized monoclonal antibody 19F3H1L1

The nucleic acid sequence of the V_{H} is as shown in SEQ ID NO: 5, and was 363 bp in length.

The amino acid sequence encoded thereby is as shown in SEQ ID NO: 6, and was 121aa in length, in which the sequence of heavy chain CDR1 is as shown in SEQ ID NO: 15, the sequence of heavy chain CDR2 is as shown in SEQ ID NO: 16, and the sequence of heavy chain CDR3 is as shown in SEQ ID NO: 17.

The nucleic acid sequence of the V_{L} is as shown in SEQ ID NO: 7, and was 339 bp in length.

The amino acid sequence encoded thereby is as shown in SEQ ID NO: 8, and was 113aa in length, in which the sequence of light chain CDR1 is as shown in SEQ ID NO: 18, the sequence of light chain CDR2 is as shown in SEQ ID NO: 19, and the sequence of light chain CDR3 is as shown in SEQ ID NO: 20.

### (2) V_{H} and V_{L} sequences of humanized monoclonal antibody 19F3H2L2

The nucleic acid sequence of the V_{H} is as shown in SEQ ID NO: 9, and was 363 bp in length.

The amino acid sequence encoded thereby is as shown in SEQ ID NO: 10, and was 121aa in length, in which the sequence of heavy chain CDR1 is as shown in SEQ ID NO: 15, the sequence of heavy chain CDR2 is as shown in SEQ ID NO: 16, and the sequence of heavy chain CDR3 is as shown in SEQ ID NO: 17.

The nucleic acid sequence of the V_{L} is as shown in SEQ ID NO: 11, and was 339 bp in length.

The amino acid sequence encoded thereby is as shown in SEQ ID NO: 12, and was 113aa in length, in which the sequence of light chain CDR1 is as shown in SEQ ID NO: 18, the sequence of light chain CDR2 is as shown in SEQ ID NO: 19, and the sequence of light chain CDR3 is as shown in SEQ ID NO: 20.

### (3) V_{H} and V_{L} sequences of humanized monoclonal antibody 19F3H2L3

The nucleic acid sequence of the V_{H} is as shown in SEQ ID NO: 9, and was 363 bp in length.

The amino acid sequence encoded thereby is as shown in SEQ ID NO: 10, and was 121aa in length, in which the sequence of heavy chain CDR1 is as shown in SEQ ID NO: 15, the sequence of heavy chain CDR2 is as shown in SEQ ID NO: 16, and the sequence of heavy chain CDR3 is as shown in SEQ ID NO: 17.

The nucleic acid sequence of the V_{L} is as shown in SEQ ID NO: 13, and was 339 bp in length.

The amino acid sequence encoded thereby is as shown in SEQ ID NO: 14, and was 113aa in length, in which the sequence of light chain CDR1 is as shown in SEQ ID NO: 18, the sequence of light chain CDR2 is as shown in SEQ ID NO: 19, and the sequence of light chain CDR3 is as shown in SEQ ID NO: 20.

### 3. Preparation of humanized antibodies 19F3H1L1, 19F3H2L2 and 19F3H2L3

Ig gamma-1 chain C region, ACCESSION: P01857 was used for the C_{H}; Ig kappa chain C region, ACCESSION: P01834 was used for the C_{L}.

19F3H1L1 heavy chain cDNA and light chain cDNA, 19F3H2L2 heavy chain cDNA and light chain cDNA, and 19F3H2L3 heavy chain cDNA and light chain cDNA were cloned into pUC57simple (provided by GenScript) vectors, respectively, to obtain pUC57simple-19F3H1, pUC57simple-19F3L1; pUC57simple-19F3H2, pUC57simple-19F3L2 and pUC57simple-19F3L3, respectively. With reference to the standard technology introduced by "Molecular Cloning: A Laboratory Manual (2nd Edition)", EcoRI & HindIII were digested and synthesized into heavy and light chain full-length genes, which were subcloned into expression vector pcDNA3.1 through the digestion of restriction enzymes (EcoRI & HindIII) to obtain expression plasmids pcDNA3.1-19F3H1, pcDNA3.1-19F3L1, pcDNA3.1-19F3H2, pcDNA3.1-19F3L2, and pcDNA3.1-19F3L3, and sequencing analysis was performed on the heavy/light chain genes of the recombinant expression plasmids. Subsequently, gene combinations containing the corresponding light and heavy chain recombinant plasmid designs (pcDNA3.1-19F3H1/pcDNA3.1-19F3L1, pcDNA3.1-19F3H2/pcDNA3.1-19F3L2, and pcDNA3.1-19F3H2/pcDNA3.1-19F3L3) were co-transfected with 293F cells, respectively, and the culture solution was harvested for purification. After passing sequencing validation, the expression plasmid of endotoxin-grade was prepared and the plasmid was transiently transfected with HEK293 cells for antibody expression. After 7 days of culture, the cell culture solution was harvested, and affinity purification was performed using Protein A column to obtain the humanized antibodies.

### 4. Preparation of humanized antibody 19F3H2L3(hG1DM)

The Ig kappa chain C region, ACCESSION: P01834 was used for the C_{L} of the 19F3H2L3(hG1DM) antibody, see SEQ ID NO: 22.

Based on the Ig gamma-1 chain C region, ACCESSION: P01857, a point mutation from leucine to alanine (L234A) was introduced at site 234, and a point mutation from leucine to alanine (L235A) was introduced at site 235 of the C_{H} to obtain a humanized antibody named 19F3H2L3(hG1DM). See SEQ ID NO: 23 for the full-length heavy chain amino acid sequence of 19F3H2L3(hG1DM) and see SEQ ID NO: 24 for the full-length light chain amino acid sequence of 19F3H2L3(hG1DM).

The heavy chain cDNA and light chain cDNA of 19F3H2L3(hG1DM) were cloned into pUC57simple (provided by GenScript) vectors, respectively, to obtain pUC57simple-19F3H2(hG1DM) and pUC57simple-19F3L3, respectively. With reference to the standard technology introduced by "Molecular Cloning: A Laboratory Manual (2nd Edition)", EcoRI & HindIII were digested and synthesized into heavy and light chain full-length genes, which were subcloned into expression vector pcDNA3.1 through the digestion of restriction enzymes (EcoRI & HindIII) to obtain expression plasmids pcDNA3.1-19F3H2(hG1DM) and pcDNA3.1-19F3L3, and sequencing analysis was performed on the heavy/light chain genes of the recombinant expression plasmids. Subsequently, the gene combinations containing light and heavy chain recombinant plasmid designs (pcDNA3.1-19F3H2(hG1DM)/pcDNA3.1-19F3L3) were co-transfected with 293F cells, respectively, and the culture solution was harvested for purification. After passing sequencing validation, the expression plasmid of endotoxin-grade was prepared and the plasmid was transiently transfected with HEK293 cells for antibody expression. After 7 days of culture, the cell culture solution was harvested, and affinity purification was performed using Protein A column to obtain the humanized antibodies.

### Example 4: Effective induction of B cell activation by anti-CD73 antibody

CD69 is a type C transmembrane glycoprotein that is often expressed on the surface of activated T, B, NK and other lymphocytes, and is typically used as a marker of early activation of lymphocytes. CD83 is another early activation marker that is widely expressed in mature DC, and also expressed in activated immune cells such as B lymphocytes. CD83 is capable of preventing the degradation of MHC class II and CD86 molecules on the cell surface and participates in immunomodulation. After B cells are activated, they may become memory B cells or plasma cells, which secrete antibody and cytokines, and participate the immune response to infection by exogenous microorganisms such as viruses.

In this test, CD69 and CD83 were used as molecular markers of B cell activation, and the MFI values and positive rates of CD69 and CD83 were directly proportional to B cell activation activity.

Experimental steps: Healthy human peripheral blood PBMCs were isolated according to the instructions for the isolation medium in the Ficoll-PaqueTM Plus kit, and the isolated PBMCs were cryopreserved for later use. On the day of the experiment, PBMCs were routinely resuscitated and resuspended in complete medium (RPMI 1640 + 10% FBS + 1% sodium pyruvate + 1% non-essential amino acid + 1% L-glutamine), inoculated into a 12-well plate at 240 ^{∗} 10⁴ cells/500 µL/well, 500 µL of the solution with the corresponding antibody concentration was added, with 2 replicate wells per group, and the wells were cultured overnight. On the next day, the cells in the wells were dispersed by pipetting, each group was divided into 5 wells, centrifuged at 750 * g for 5 min, the cells were harvested, 100 µL of mouse IgG isotype control antibody (final concentration of 5 µg/mL) was added to each well, incubated on ice for 20 min, and centrifuged at 750 * g for 5 min. The supernatant was removed, and 100 µL of the following secondary antibodies were added to each corresponding well of each group, respectively: FITC-labeled anti-human CD3 antibody (diluted 50 folds), PE-labeled anti-human CD19 antibody (diluted 50 folds), APC-labeled anti-human CD69 antibody (diluted 100 folds), APC-labeled anti-human CD83 antibody (diluted 50 folds), APC-labeled mouse IgG1 isotype control antibody (diluted 100 folds), incubated on ice and protected from light for 40 min, 100 µL of 1% PBSA (PBS containing 1% BSA) was added to each well, centrifugation was performed at 750 * g for 5 min, and the supernatant was removed. Cells were resuspended with 200 µL/tube of 1% PBSA, precipitated, transferred to flow tubes, and loaded to a flow cytometer (FACS Calibur) for detection.

Flowing software was used to plot flow histograms and scatter plots, and the MFI values expressed by CD69 and CD83 in CD3-CD19+ cell subsets were analyzed. Graphpad prism software was used to plot the mean ± standard deviation SD according to the corresponding MFI values and perform one-way analysis of variance. P < 0.05 showed that the difference was significant, and P < 0.01 showed that the difference was very significant.

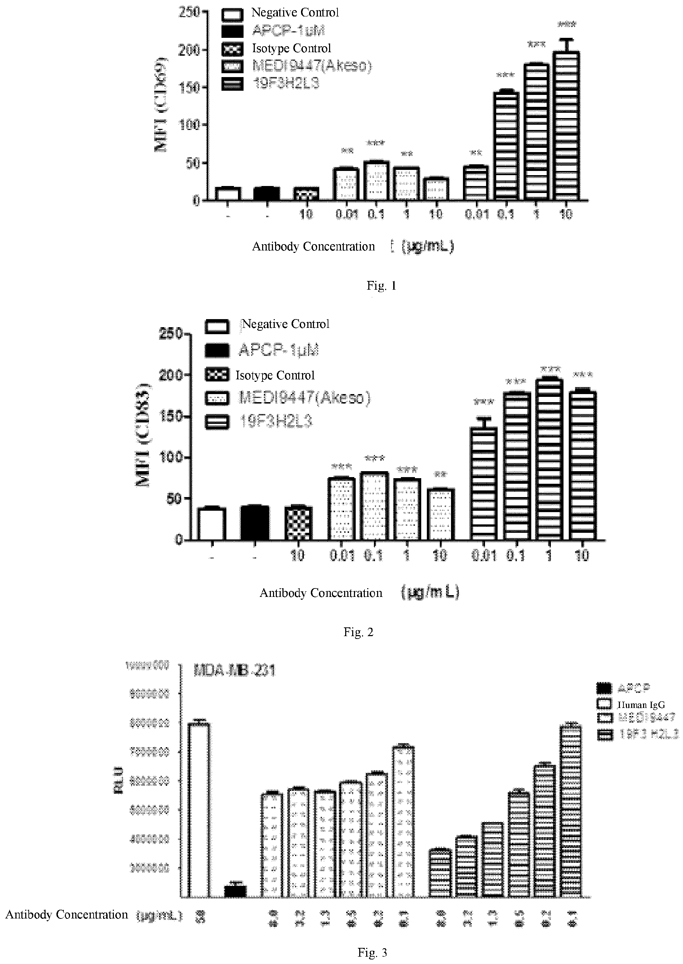

The results are shown in Figs. 1 and 2. The MFI values of CD69 and CD83 in the 19F3H2L3 treatment group were significantly higher than that of the isotype control, indicating that 19F3H2L3 is capable of inducing B cell activation and upregulating the expression of CD69 and CD83, and the activity is better than that of the same-target control antibody MEDI9447 under clinical research. However, CD73 enzyme activity inhibitor APCP (Sigma, catalog number: M3763-10MG) had no effect on the expression of CD69 and CD83 on the surface of B cells, indicating that 19F3H2L3 is capable of inducing activation of B cells through signaling pathways different from CD73 enzyme activity inhibition.

The detection results of this test showed that 19F3H2L3 is capable of significantly upregulating the expression of CD69 and CD83 on the surface of B cells (CD3⁻CD19⁺), indicating that 19F3H2L3 has the biological activity of inducing B cell activation, and this activity is significantly better than the same-target control antibody MEDI9447 under clinical research. At the same time, the detection results showed that the CD73 enzyme activity inhibitor APCP had no effect on the expression of CD69 and CD83 on the surface of B cells, indicating that 19F3H2L3 is capable of inducing the activation of B cells through signaling pathways different from CD73 enzyme activity inhibition, thereby promoting the immune response to viral antigens. Antibody 19F3, antibody 19F3H1L1, and antibody 19F3H2L2 achieved similar results.

### Example 5: Detection of inhibition of enzyme activity of endogenously expressed CD73 by anti-CD73 antibody

### 1. Detection of inhibitory activity of enzyme activity of endogenously expressed CD73 in MDA-MB-231 cells by anti-CD73 antibody

The experimental steps are as follows: log-phase MDA-MB-231 cells in good condition were taken, resuspended in serum-free RPMI-1640 medium, and counted; MDA-MB-231 cells were inoculated in a 96-well plate, at 3 * 10⁴ cells/100 uL/well; the antibody was diluted with serum-free RPMI-1640 medium, and 2.5-fold gradient dilution was performed at the initial concentration of 200 µg/mL; the antibody was added to a 96-well plate, at 50 uL/well, and incubated for 1 hour at 37°C. After 1 hour, 50 uL of RPMI-1640-diluted 600 µM AMP was added to each well; 25 uL of cell culture supernatant was taken 3 hours later, transferred to a new 96-well plate, and 25 uL of 100 µM ATP was added to each well; 50 uL of CTG (CellTiter-Glo^{®} One Solution Assay, promega, Cat: G8461) chromogen solution was used to develop the color, and the data was read by a multi-label microplate detector (PerkinElmer 2140-0020).

Experimental results: The results are shown in Fig. 3. Both 19F3H2L3 and the same-target positive control drug MEDI9447 were capable of inhibiting the enzyme activity of CD73 endogenously expressed in MDA-MB-231 that catalyzes AMP to adenosine in a dose-dependent manner, thereby reducing the mean fluorescence intensity (MFI) RLU (relative light unit) produced in a dose-dependent manner.

The above experimental results indicate that the added AMP may be converted to adenosine under the enzyme catalysis of CD73 endogenously expressed on the cell surface of MDA-MB-231 in the absence of CD73 antibody treatment, thereby relieving the inhibition of luciferase activity. After the addition of the antibody, CD73 is bound by the antibody, which reduces the enzyme catalytic activity thereof and prevents the conversion of AMP to adenosine. This suggests that the anti-CD73 antibody effectively inhibits the enzyme activity of CD73 in a non-substrate competitive manner and reduces the production of adenosine.

### 2. Detection of enzyme activity of anti-CD73 antibody added to U87-MG cells

The experimental steps are as follows: log-phase U87-MG cells in good condition were taken, resuspended in serum-free RPMI-1640 medium, and counted; U87-MG cells were inoculated to a 96-well plate, at 3 ^{∗} 10⁴ cells/100 µL/well; the antibody was diluted with serum-free RPMI-1640 medium, 2.5-fold gradient dilution was performed at the initial concentration of 200 µg/mL; the antibody was added to a 96-well plate, at 50 µL per well, and incubated for 1 hour at 37°C. After 1 hour, 50 uL of RPMI-1640-diluted 600 µM AMP was added to each well; 25 uL of cell culture supernatant was taken 3 hours later, transferred to a new 96-well plate, and 25 uL of 100 µM ATP was added to each well; 50 uL of CTG (CellTiter-Glo^{®} One Solution Assay, promega, Cat: G8461) chromogen solution was used to develop the color, and the data was read by a multi-label microplate detector (PerkinElmer 2140-0020).

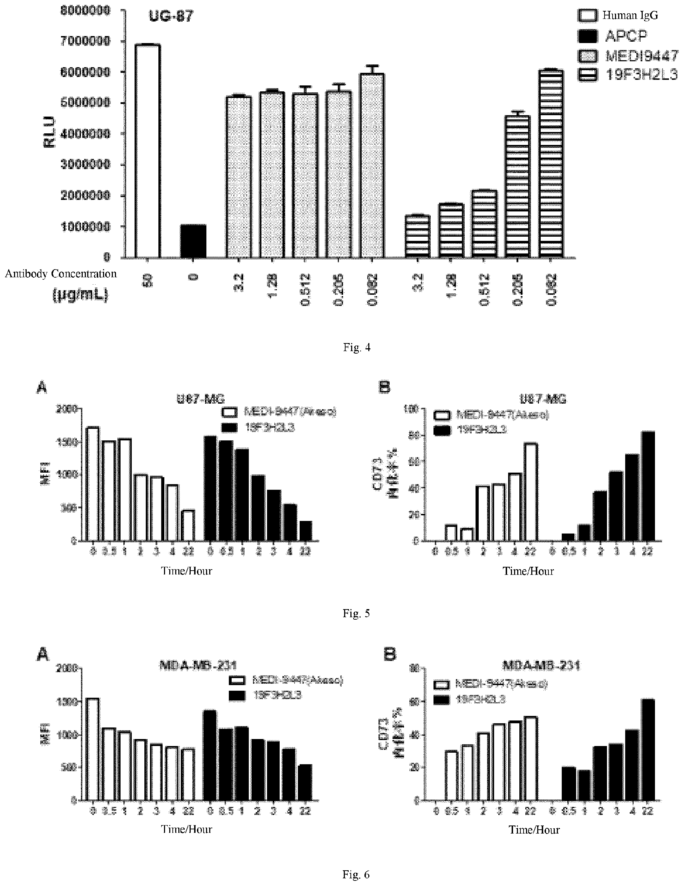

Experimental results: The results are shown in Fig. 4. Both 19F3H2L3 and the same-target positive control drug MEDI9447 were capable of inhibiting the enzyme activity of CD73 endogenously expressed in MDA-MB-231 that catalyzes AMP to adenosine in a dose-dependent manner, thereby reducing the MFI RLU produced in a dose-dependent manner.

The above experimental results indicate that the added AMP may be converted to adenosine under the enzyme catalysis of CD73 endogenously expressed on the cell surface of 87-MG in the absence of CD73 antibody treatment, thereby relieving the inhibition of luciferase activity. After the addition of the antibody, CD73 is bound by the antibody, which reduces the enzyme catalytic activity thereof and prevents the conversion of AMP to adenosine. This suggests that the anti-CD73 antibody effectively inhibits the enzyme activity of CD73 in a non-substrate competitive manner and reduces the production of adenosine. Antibody 19F3, antibody 19F3H1L1, and antibody 19F3H2L2 achieved similar results.

### Example 6: Effective induction of internalization of CD73 expression on cell membrane surface by anti-CD73 antibody

In this test, the FACS method was used to evaluate the biological activity of 19F3H2L3-mediated internalization of CD73 on the surface of MDA-MB-231 cell and U87-MG cell membranes. MFI values of the indirect internalization method were inversely proportional to endocytosis activity.

Experimental steps: cells were cultured to log phase with complete medium, centrifuged at 170 × g for 5 min, the cells were harvested, counted, and viability was determined, with 30 w/sample. The antibody solution diluted to the corresponding concentration was added according to the experimental design, at 100 µL per tube, the blank and isotype control were designed, and incubated on ice for 1 h; 1,000 µL of 1% PBSA was added, centrifugation was performed at 1,200 × g for 5 min, the supernatant was discarded and washing was repeated once. 200 µL of the corresponding complete medium was added to each tube and endocytosis was performed at 37°C for different periods of time. After endocytosis, 1,000 µL of 1% PBSA (containing 0.05% sodium azide, SIGMA, catalog number: S2002-25G) was added, and centrifugation was performed at 1200 × g for 5 min. 100 µL of the corresponding fluorescent secondary antibody was added to each tube, mixed well and incubated on ice for 40 minutes away from light. 1,000 µL of 1% PBSA (containing 0.05% sodium azide) was added, centrifugation was performed at 1,200 × g for 5 min, and the supernatant was discarded. 200 µL of 1% PBSA (containing 0.05% sodium azide)/tube was added, the cells were resuspended, transferred to flow cytometry sample loading tubes, and loaded for testing.

The internalization results are shown in Figs. 5 and 6. After incubation with MDA-MB-231 and U87-MG cells for 0.5 hours at 37°C, a decrease in MFI value of 19F3H2L3 was clearly observed (see Figs. 5A and 6A), indicating that 19F3H2L3 is only capable of rapidly mediating the internalization of CD73 on the cell membrane surface. After 22 hours of incubation, the 19F3H2L3-mediated internalization rate in MDA-MB-231 cells could reach 60.75%, and the internalization rate of the same-target control drug MEDI9447 was 50.53%, which was 10.22% lower than that of 19F3H2L3 (see Fig. 6B); after 22 hours, the 19F3H2L3-mediated internalization rate of CD73 in U87-MG cells was 82.39%, and the 22-hour internalization rate of the same-target control antibody MEDI9447 was 73.65%, which was 8.74% lower than that of 19F3H2L3 (see Fig. 5B). Antibody 19F3, antibody 19F3H1L1, and antibody 19F3H2L2 achieved similar results.

### Example 7: Binding activity of anti-CD73 antibody to antigen human NT5E-Biotin as determined by ELISA

Experimental steps: After coating the microplate with streptavidin at 2 µg/mL, the microplate was incubated at 4°C for 12 hours. After the end of incubation, the antigen-coated microplate was rinsed once using PBST, and then blocked for 2 hours using 1% BSAPBST solution as the microplate blocking buffer. After the end of blocking, the microplate was washed three times using PBST. Antigen human NT5E-Biotin (prepared by Akeso Biopharma Co., Ltd., batch number: 20160505) was added at 0.5 µg/mL, incubated at 37 °C for 30 minutes, and the plate was washed three times using PBST. The antibody serially diluted with PBST solution was added to the microplate wells. See Table 1 for details on the antibody dilution gradient. The microplate with the antibody to be tested was incubated at 37°C for 30 minutes, and the microplate was washed three times using PBST after the incubation was completed. After the plate was washed, HRP-labeled goat anti-human IgG (H+L)(Jackson, catalog number: 109-035-088) diluted at a ratio of 1:5,000 or HRP-labeled goat anti-mouse IgG (H+L)(Jackson, catalog number: 115-035-062) secondary antibody working solution diluted at a ratio of 1:5,000 was added, and incubated at 37°C for 30 minutes. After the incubation was completed, the plate was washed four times using PBST, and TMB (Neogen, 308177) was added, the color developed for 5 min away from light, and the chromogenic reaction was terminated by adding the stop solution. The microplate was immediately placed into the microplate reader, and the 450 nm optical wavelength was selected to read the OD value of each well of the microplate. Data were analyzed and processed using SoftMax Pro 6.2.1 software.

See Table 1 for the OD values of each dose for detecting the binding of the anti-CD73 antibody to the antigen human NT5E-Biotin. Curve fitting was performed using the antibody concentration as the abscissa and the absorbance value as the ordinate, and the binding EC₅₀ of the antibody was calculated. The results are shown in Table 1 below.

The experimental results showed that antibodies 19F3H1L1, 19F3H2L2, 19F3H2L3 and murine antibody 19F3 could effectively bind to human NT5E-Biotin, and the binding efficiency was dose-dependent. Under basically the same experimental conditions, the EC₅₀ of the binding of 19F3H1L1 to human NT5E-Biotin was 0.049 nM, the EC₅₀ of the binding of 19F3H2L2 to human NT5E-Biotin was 0.064 nM, the EC₅₀ of the binding of 19F3 H2L3 to human NT5E-Biotin was 0.061 nM, the EC ₅₀ of the binding of the same-target positive drug MEDI9447 to human NT5E-Biotin was 0.048 nM, and the EC₅₀ of the binding of murine antibody 19F3 to human NT5E-Biotin was 0.018 nM.

The above experimental results showed that the binding activity of 19F3H1L1, 19F3H2L2, 19F3 H2L3 and murine antibody 19F3 to human NT5E-Biotin under the same experimental conditions was comparable to that of the same-target positive drug MEDI9447, respectively, suggesting that 19F3H1L1, 19F3H2L2 and 19F3H2L3 have the function of effectively binding CD73.

**Table 1 Detection Result of Binding Activity of 19F3H1L1, 19F3H2L2, 19F3H2L3 and Murine 19F3 to HNT5E-Biotin**

| **Antibody Dilution (µg/mL)** | **Antigen Coating: SA (2 µg/mL)** | | | | | | | | | | | |
|---|---|---|---|---|---|---|---|---|---|---|---|---|
| | **Human NT5E-Biotin (0.5 µg/mL)** | | | | | | | | | | | |
| | **19F3H1L1** | | **19F3H2L2** | | | **19F3H2L3** | | | **MEDI9447** | | **19F3** | |
| 0.333 | 2.648 | 2.640 | 2.598 | | 2.688 | 2.623 | | 2.588 | 2.548 | 2.527 | 2.706 | 2.743 |
| 1:3 | 2.601 | 2.697 | 2.578 | | 2.618 | 2.581 | | 2.582 | 2.573 | 2.604 | 2.736 | 2.763 |
| 1:9 | 2.407 | 2.332 | 2.163 | | 2.330 | 2.186 | | 2.257 | 2.268 | 2.284 | 2.566 | 2.641 |
| 1:27 | 1.821 | 1.820 | 1.579 | | 1.680 | 1.626 | | 1.649 | 1.774 | 1.742 | 2.330 | 2.361 |
| 1:81 | 1.044 | 1.035 | 0.870 | | 0.933 | 0.918 | | 0.931 | 1.058 | 1.030 | 1.693 | 1.769 |
| 1:243 | 0.525 | 0.516 | 0.434 | | 0.454 | 0.450 | | 0.457 | 0.536 | 0.528 | 1.001 | 1.000 |
| 1:729 | 0.260 | 0.273 | 0.239 | | 0.247 | 0.241 | | 0.252 | 0.272 | 0.275 | 0.446 | 0.470 |
| 0 | 0.125 | 0.123 | 0.119 | | 0.123 | 0.120 | | 0.123 | 0.121 | 0.116 | 0.060 | 0.062 |
| Secondary antibody | HRP-labeled goat anti-human IgG (H+L) (1:5,000) | | | | | | | | | | HRP-labeled goat anti-mouse IgG (H+L) (1:5,000) | |
| EC₅₀ (nM) | 0.049 | | | 0.064 | | | 0.061 | | 0.048 | | 0.018 | |

### Example 8: Effective induction of production of antibodies against COVID-19 S protein by anti-CD73 antibody

### 1. Experimental materials:

hIgG1DM, manufactured by Akeso Biopharma Co., Ltd.; S-protein (nCoV _SRBD-mFc, refer to YP_009724390.1 for the sequence), manufactured by Akeso Biopharma Co., Ltd.; CFA (Complete Freund's Adjuvant), purchased from SIGMA, catalog number: F5881-10ml; IFA (Incomplete Freund's Adjuvant), purchased from SIGMA, catalog number: F5506-10ml.

### 2. Experimental method:

The experimental protocol and dosing regimen are shown in the table below.

**Table 2. Experimental Protocol for Effective Induction of Production of Antibodies against COVID-19 S Proteins by Anti-CD73 Antibody**

| **Group** | **Experimental Protocol** | **Dosing Regimen** |
|---|---|---|
| **Model Group** | After adequate emulsification of S protein and CFA on D0, administer a subcutaneous injection (two-point injection, lower extremities and inguinal region of mice), at 25 µg/mouse; | Saline, IP, BIW × 6, start administration the day after immunization |
| **High-dose isotype control** | | hIgG1DM, 50 mg/kg, IP, BIW × 6, start administration the day after immunization |
| **Low-dose isotype control** | | hIgG1DM, 10 mg/kg, IP, BIW × 6, start administration the day after immunization |
| | administer booster immunization (S-protein and IFA) on D14 and D43 via subcutaneous injection, at 25 µg/mouse | |
| 19F3H2L3(hG1DM) **High-dose** | | 19F3H2L3(hG1DM), 50 mg/kg, IP, BIW × 6, start administration the day after immunization |
| **Low-dose** 19F3H2L3(hG1DM) | | 19F3H2L3(hG1DM), 10 mg/kg, IP, BIW × 6, start administration the day after immunization |

| | | |
|---|---|---|
| Note: IP: Intraperitoneal injection; BIW * 6: administration twice a week for a total of 6 administrations | | |

### 3. Experimental results:

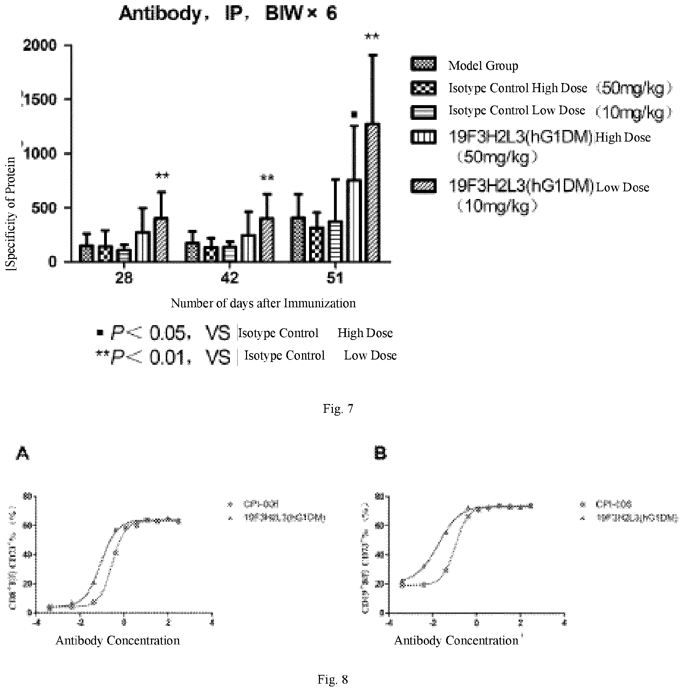

As shown in Fig. 7. In the model group, after mice received S protein (25 µg/mouse) + adjuvant-induced injection, the level of S protein-specific IgG in mice serum increased, suggesting that S protein (25 µg/mouse) + adjuvant could effectively induce the body's immune response against COVID-19 S protein. S protein-specific IgG in the serum of mice in the 19F3H2L3(hG1DM) administration group increased at varying degrees compared with that in the isotype control group. On Day 51, the difference between the high- and low-dose 19F3H2L3(hG1DM) groups was statistically significant (T-test), and the promotion effect of S-protein-specific IgG in mice was more significant in the low-dose 19F3H2L3(hG1DM) (10 mg/kg) group. Antibody 19F3, antibody 19F3H1L1, and antibody 19F3H2L2 achieved similar results.

### Example 9: Detection of binding activity of anti-CD73 antibody 19F3H2L3(hG1DM) to CD8+ T-cells and CD19+ B cells by FACS

The experimental steps are as follows: The PBMCs were resuscitated, centrifuged at 1,200 rpm for 5 min, and the supernatant was discarded, resuspended in complete medium, counted, and incubated overnight at 37°C. The solution was centrifuged at 1,200 rpm for 5 min, cells were harvested, diluted antibody solution was added at 100 µL/well, mixed well, and incubated in a refrigerator at 4°C for 1 hour. The solution was centrifuged at 750 × g for 5 min, the supernatant was discarded, and washed 2 times with 200 µL of 1X PBS (containing 1% BSA and 0.05% NaN3). Anti-human CD3-FITC (2 µL/100 µL), anti-human CD19-PE (2 µL/100 µL) (Biolegend, article number: 302254), anti-human CD8-PerCP Cy5.5 (1 µL/100 µL), anti-human IgG Fc-AF647 (1:300) (Southern Biotech, catalog number: 9040-31) were added, and incubated at 4°C for 30 minutes. The solution was centrifuged at 750 × g for 5 min, the supernatant was discarded, washed once with 200 µL of 1X PBS (including 1% BSA and 0.05% NaN3); resuspended with 300 µL of 1X PBS (including 1% BSA and 0.05% NaN3), transferred to flow tubes, and loaded for testing.

The results are shown in Figs. 8 and 3. The binding activity EC50 (nM) of anti-CD73 antibody 19F3H2L3(hG1DM) and positive control antibody CPI-006 to CD8+T-cells was 0.087 nM and 0.288 nM, respectively, the binding activity EC50 (nM) of anti-CD73 antibody 19F3H2L3(hG1DM) and positive control antibody CPI-006 to CD19+B cells were 0.018 nM and 0.108 nM, respectively. Both 19F3H2L3(hG1DM) and the same-target positive control CPI-006 bound to CD73 expressed by human CD8⁺ T and CD19⁺ B cells in a dose-dependent manner, thereby increasing the MFI in a dose-dependent manner.

The above results indicate that under the same experimental conditions, 19F3H2L3(hG1DM) binds to CD8+ T-cells and CD19+ B cells more strongly than the positive control antibody CPI-006.

**Table 3: Detection of Binding Activity of Anti-CD73 Antibody to CD8+ T Cells and CD19+ B Cells by FACS**

| Concentration (nM) | CD73+percent% of CD8+T | | CD73+percent% of CD 19+B | |
|---|---|---|---|---|
| | CPI-006 | 19F3H2L3(hG1DM) | CPI-006 | 19F3H2L3(hG1DM) |
| 0 | 0.67 | 0.67 | 0.00 | 0.00 |
| 0.00041 | 4.02 | 3.08 | 19.10 | 22.30 |
| 0.0041 | 4.12 | 7.10 | 19.30 | 32.70 |
| 0.041 | 7.25 | 20.80 | 29.40 | 55.60 |
| 0.41 | 41.10 | 57.40 | 66.30 | 72.80 |
| 1.23 | 58.50 | 58.40 | 71.90 | 70.80 |
| 3.70 | 59.80 | 62.60 | 71.90 | 73.00 |
| 11.11 | 63.80 | 64.00 | 73.30 | 74.20 |
| 33.33 | 63.20 | 63.20 | 72.50 | 73.70 |
| 100 | 64.40 | 65.00 | 72.70 | 72.60 |
| 300 | 62.40 | 64.10 | 73.70 | 73.30 |
| EC50 (nM) | 0.288 | 0.087 | 0.108 | 0.018 |

### Example 10: Inhibition of enzyme activity of CD73 expressed by human PBMC membrane by anti-CD73 antibody 19F3H2L3(hG1DM)

The experimental steps are as follows: fresh human PBMCs were isolated by density gradient centrifugation and resuspended in assay medium (serum-free RPMI-1640). The number of cells was counted and viability was determined, and the solution was inoculated in a 96-well plate at 60 µL/well based on 10 ^{∗} 10⁴ cells/well. The antibody was diluted using assay medium to the following concentrations: 30, 10, 3.3, 1.1, 0.11, 0.011, 0.0011, and 0.00011 µg/mL. The antibody was added to a 96-well plate, at 60 µL/well, mixed gently with the cells, and incubated at 37°C for 30 minutes. An AMP solution with a concentration of 60 µM (TCI, Cat. A0158) was prepared using assay medium for later use; ATP solution with a concentration of 30 µM was prepared using TM buffer (akesobio, 20191009) for later use. After incubating the cell antibody for 30 min, the prepared AMP solution was added, at 60 µL/well, mixed gently, and incubated overnight at 37°C (10 h). Before the end of overnight incubation, a clean black 96-well plate was taken out, CTG (promega, Cat: G8461) was added, at 40 µL/well; after the end of overnight incubation, the 96-well plate in the incubator was taken out, centrifuged at 750 × g for 5 min, and the supernatant was transferred to the black 96-well plate with CTG added at 100 µL per well, the plate was tapped gently, and placed at room temperature away from light for 5 min. After 5 min, the prepared ATP solution was added to the black 96-well plate, at 10 µL/well, the plate was tapped gently and mixed well, and placed at room temperature away from light for 15 min. After 15 minutes, the RLU value was detected using the Envision microplate reader.

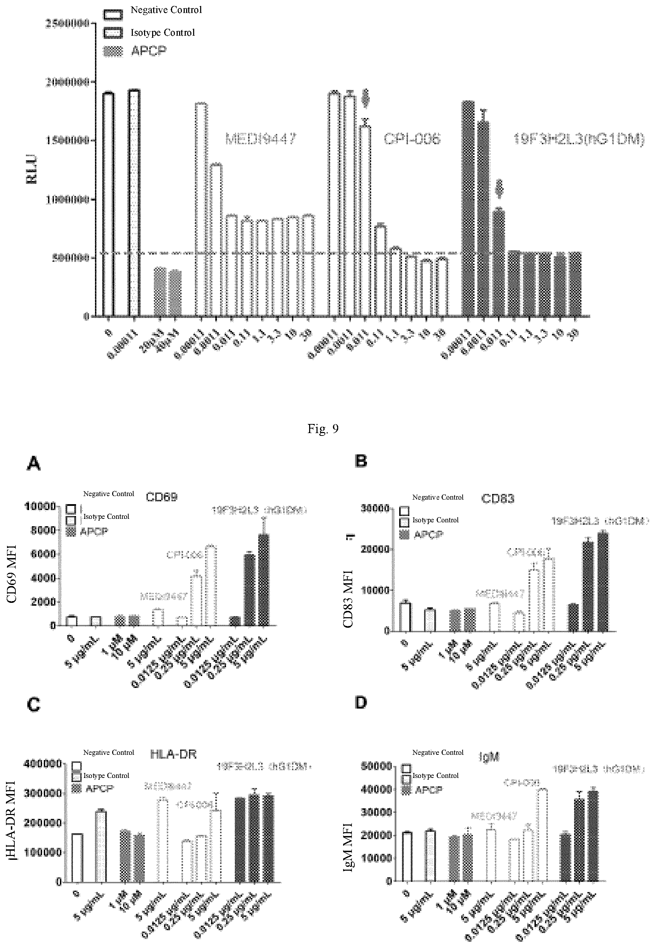

Experimental results: The results are shown in Fig. 9 and Table 4. The enzyme activity and effectiveness EC50 (nM) of 19F3H2L3(hG1DM), MEDI9447 and CPI-006 in the inhibition of CD73 expressed by human PBMC membrane was 0.033 nM, 0.0064 nM and 0.2258 nM, respectively. Both 19F3H2L3(hG1DM) and same-target positive control drugs MEDI9447 and CPI-006 inhibited the enzyme activity of CD73 expressed by human PBMCs that catalyzes AMP into adenosine in a dose-dependent manner, thereby reducing the MFI produced in a dose-dependent manner. In addition, the inhibition rate of anti-CD73 antibody 19F3H2L3(hG1DM) was greater than that of the positive control drugs MEDI9447 and CPI-006.

**Table 4: Inhibition of Enzyme Activity of CD73 Expressed by Human PBMC Membrane by Anti-CD73 Antibody**

| Antibody Name | MEDI9447 | CPI-006 | 19F3H2L3(hG1DM) |
|---|---|---|---|
| Inhibition rate EC50 (nM) | 0.0064 | 0.2258 | 0.0330 |
| R2 | 0.9984 | 0.9986 | 0.9997 |

### Example 11: Effective induction of B cell activation by anti-CD73 antibody 19F3H2L3(hG1DM) as detected by FACS

CD69 is a type C transmembrane glycoprotein that is often expressed on the surface of activated T, B, NK and other lymphocytes, and is typically used as a marker of early activation of lymphocytes. CD83 is another early activation marker that is widely expressed in mature DC, and also expressed in activated immune cells such as B lymphocytes. CD83 is capable of preventing the degradation of MHC class II and CD86 molecules on the cell surface and participates in immunomodulation. After B cells are activated, they may become memory B cells or plasma cells, which secrete antibody and cytokines, and participate the immune response to infection by exogenous microorganisms such as viruses. Activated B cells produce IgM-based antibodies. HLA-DR is an MHC class II molecule that is expressed on B cells, and increased HLA-DR expression is a marker of B lymphocyte activation. Therefore, in this test, CD69, CD83, HLA-DR and IgM were used as molecular markers of B cell activation, and the MFI values and positive rates of CD69, CD83, HLA-DR and IgM were directly proportional to B cell activation activity.

The experimental steps are as follows:
B cells were resuscitated, resuspended in complete medium, counted, inoculated in 96-well plates at 100,000 cells/100 µL/well, and two replicate wells were set in each group; antibodies or APCP (adenosine 5'-(α,β-methylene)diphosphate, Sigma, catalog number: M3763-10MG) were added, mixed well, and incubated in an incubator; experimental wells (added with 50 µL of hIgG1 (Akesobio, batch number: 20170424) were first incubated with B cells for 30 min, and then 50 µL of solution with the corresponding antibody concentration was added). After incubation for 4 h and 3 days, respectively, B cells were harvested and flow cytometry was performed to detect the CD69/CD83/HLA-DR/IgM expression level of CD3-CD19+ B cells.

FACS operating procedures: the B cells in each well of the well plate were gently mixed, transferred equally to 5 wells of the 96-well plate, centrifuged at 750 × g for 5 min, the supernatant was removed, resuspended in PBSA (PBS containing 1% BSA), and mouse IgG (Mouse IgG Isotype Control) was added at 100 uL/well to obtain a final concentration of 5 µg/ml. The solution was incubated on ice for 20 min, centrifuged at 750 × g for 5 min, the supernatant was discarded, and Mix1/2/3/4/5/6/7 was added, respectively.
Mix1: 100 uL of PBSA;
Mix2: 100 µL of PBSA contains 2 µL of FITC anti-human CD3 (purchased from Biolegend, catalog number: 344804) + 2 µL of PE anti-human CD19 (purchased from Biolegend, catalog number: 302254);
Mix3: 100 µL of PBSA contains 2 µL of FITC anti-human CD3 + 2 µL of PE anti-human CD19 + 1 µL of APC anti-human CD69 (purchased from Biolegend, catalog number: 310910);
Mix4: 100 µL of PBSA contains 2 µL of FITC anti-human CD3 + 2 µL of PE anti-human CD19 + 2 µL of APC anti-human CD83 (purchased from Biolegend, catalog number: 305312);
Mix5: 100 µL of PBSA contains 2 µL of FITC anti-human CD3 + 2 µL of PE anti-human CD19 + 2 µL of APC anti-human HLA-DR (purchased from Biolegend, catalog number: 361610);
Mix6: 100 µL of PBSA contains 2 µL of FITC anti-human CD3 + 2 µL of PE anti-human CD19 + 2 µL of APC anti-human IgD (purchased from Biolegend, catalog number: 348222) + 2 µL of PerCP/Cyanine 5.5 anti-human CD27 antibody (Biolegend, catalog number: 356408);

Mix7: 100 µL of PBSA contains 2 µL of FITC anti-human CD3 + 2 µL of PE anti-human CD19 + 2 µL of APC anti-human IgM (purchased from Biolegend, catalog number: 314510).

The solution was incubated on ice for 40 min, 200 µL of PBSA/well was added, centrifuged at 750 × g for 5 min, and washed; 200 µL of PBSA/well was added, the cells were resuspended and loaded for testing.

The results are shown in Fig. 10. The MFI values of CD69, CD83, HLA-DR, and IgM in the 19F3H2L3(hG1DM) treatment group were significantly higher than that of the isotype control, indicating that 19F3H2L3(hG1DM) is capable of inducing B cell activation and upregulating the expression of CD69, CD83, HLA-DR, and IgM and the activity is better than that of the same-target control antibodies MEDI9447 and CPI-006 under clinical research. However, CD73 enzyme activity inhibitor APCP (Sigma, catalog number: M3763-10MG) had no effect on the expression of CD69, CD83, HLA-DR, and IgM on the surface of B cells, indicating that 19F3H2L3(hG1DM) is capable of inducing activation of B cells through signaling pathways different from CD73 enzyme inhibition.

### Example 12: Stimulation of B cell proliferation by anti-CD73 antibody 19F3H2L3(hG1DM)

The experimental steps are as follows: PBMCs were resuscitated, centrifuged at 250 × g for 5 min, resuspended in complete medium and counted. The solution was centrifuged, the complete medium was discarded, washed once with RPMI-1640 and resuspended. 0.25 µM CFSE (Biolegend, catalog number: 423801) solution was added to 1 × 10⁶ cells/mL, incubated in an incubator at 37°C for 2 min away from light; 2- to 3-fold serum-containing medium was added to terminate the reaction, the solution was centrifuged at 250 × g (1,200 rpm) for 5 min, and the supernatant was discarded; the cells were resuspended in RPMI-1640 complete medium, counted, the cell suspension was added to a 24-well plate at 2.4 ^{∗} 10 ⁶ cells/well, the corresponding concentration of antibody solution was added to each well according to the experimental design, and incubated in a carbon dioxide incubator at 37°C and 5% CO ₂ for 7 days. Half of the supernatant in the 24-well plate was discarded on D3, and the corresponding concentration of antibody solution diluted with complete medium was added. On D6 of the culture, half of the supernatant in the 24-well plate was discarded, and the corresponding concentration of antibody solution diluted with complete medium was added. On D7 of the culture, the cells were harvested from the 24-well plate and loaded onto the flow cytometer according to the following steps. The cells were centrifuged at 1,200 × g for 5 min, harvested, the corresponding cell number group was obtained and incubated with 5 µg/mL MS mouse IgG (Thermofisher, catalog number: 10400C) for 20 min; the solution was centrifuged, washed, and the corresponding fluorescent antibody was added for incubation for 60 min. The solution was resuspended in 1% PBSA, transferred to flow tubes, and loaded for testing.

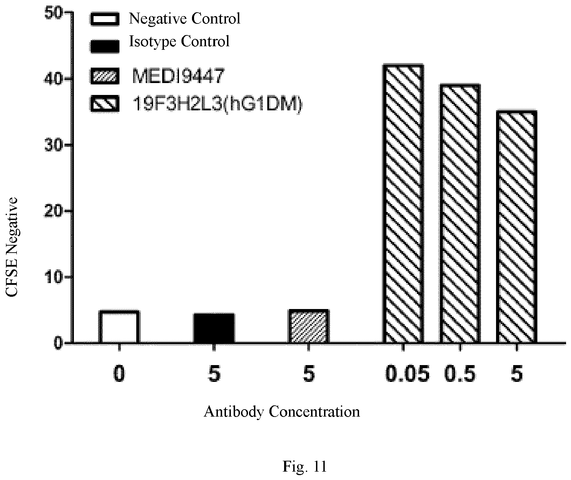

The results are shown in Fig. 11. After the different antibodies were added to human PBMC and treated for 7 days, no B cell proliferation was observed in the MEDI9447-treated group compared with human IgG1DM, while B cell proliferation could be significantly promoted by 19F3H2L3 with a concentration as low as 0.05 µg/mL. Antibody 19F3, antibody 19F3H1L1, and antibody 19F3H2L2 achieved similar results.

### Sequence List

Akeso Biopharma Co., Ltd.
Title of Invention: Anti-CD73 Antibody and Use Thereof
Nucleic acid sequence of 19F3 V_{H} (SEQ ID NO: 1) 
GAGGTGCAGCTGCAGCAGTCCGGACCAGAGCTGGTGAAGCCTGGCGCCTCCATGCGGAT
GTCTTGTAAGGCCTCTGGCTACAGCTTCACCGGCTATACAATGAACTGGGTGAAGCAGTC
TCACGGCAAGAATCTGGAGTGGATCGGCCTGATCAACCCTTACAATGCCGGCACCAGCTA
TAACCAGAAGTTTAAGGGCAAGGCCACCCTGACAGTGGACAAGAGCTCCTCTACCGCCT
ACATGGAGCTGCTGTCCCTGACATCTGAGGATAGCGCCGTGTACTATTGCGCCCGGTCCG
AGTACAGATATGGCGGCGACTACTTTGATTATTGGGGCCAGGGCACCACACTGACAGTGA
GCTCC
Amino acid sequence of 19F3 V_{H} (SEQ ID NO: 2) 
EVQLQQSGPELVKPGASMRMSCKASGYSFTGYTMNWVKQSHGKNLEWIGLINPYNAGTSY
NQKFKGKATLTVDKSSSTAYMELLSLTSEDSAVYYCARSEYRYGGDYFDYWGQGTTLTVSS
Nucleic acid sequence of 19F3 V_{L}: (SEQ ID NO: 3) 
GACATCGTGATGACCCAGTCTCCAAGCTCCCTGGCAATGAGCGTGGGACAGAAGGTGAC
AATGTCTTGTAAGTCTAGCCAGAGCCTGCTGAACTCCTCTAATCAGAAGAACTACCTGGC
CTGGTATCAGCAGAAGCCAGGCCAGTCTCCCAAGCTGCTGGTGTACTTTGCCAGCACCA
GGGAGTCCGGAGTGCCTGACAGATTCATCGGCTCCGGCTCTGGCACAGACTTCACCCTG
ACAATCAGCTCCGTGCAGGCAGAGGACCTGGCAGATTATTTCTGCCAGCAGCACTACGA
CACCCCTTATACATTTGGCGGCGGCACCAAGCTGGAGATCAAG
Amino acid sequence of 19F3 V_{L}: (SEQ ID NO: 4) 
DIVMTQSPSSLAMSVGQKVTMSCKSSQSLLNSSNQKNYLAWYQQKPGQSPKLLVYFASTRE
SGVPDRFIGSGSGTDFTLTISSVQAEDLADYFCQQHYDTPYTFGGGTKLEIK
Nucleic acid sequence of 19F3H1L1 V_{H}: (SEQ ID NO: 5) 
CAGGTGCAGCTGCAGCAGTCTGGAGCAGAGGTGGTGAAGCCAGGAGCCTCTATGAAGA
TGAGCTGTAAGGCCAGCGGCTACTCCTTCACCGGCTATACAATGAACTGGGTGAAGCAG
GCCCACGGCCAGAATCTGGAGTGGATCGGCCTGATCAACCCTTACAATGCCGGCACCTC
 
TTATAACCAGAAGTTTCAGGGCAAGGCCACCCTGACAGTGGACAAGTCCACCTCTACAG
CCTACATGGAGCTGAGCTCCCTGCGGAGCGAGGATACAGCCGTGTACTATTGCGCCCGG
TCCGAGTACAGATATGGCGGCGACTACTTTGATTATTGGGGCCAGGGCACCACACTGAC
CGTGTCTAGC
Amino acid sequence of 19F3H1L1 V_{H}: (SEQ ID NO: 6) 
QVQLQQSGAEVVKPGASMKMSCKASGYSFTGYTMNWVKQAHGQNLEWIGLINPYNAGTS
YNQKFQGKATLTVDKSTSTAYMELSSLRSEDTAVYYCARSEYRYGGDYFDYWGQGTTLTV
SS
Nucleic acid sequence of 19F3H1L1 V_{L}: (SEQ ID NO: 7) 
GACATCGTGATGACCCAGTCCCCAAGCTCCCTGGCAATGTCTGTGGGAGAGAGGGTGAC
AATGTCCTGTAAGTCTAGCCAGTCTCTGCTGAACTCCTCTAATCAGAAGAACTACCTGG
CCTGGTATCAGCAGAAGCCCGGCCAGGCCCCTAAGCTGCTGGTGTACTTTGCCTCTACC
AGGGAGAGCGGAGTGCCAGACAGATTCTCTGGCAGCGGCTCCGGCACAGACTTCACCC
TGACAATCAGCTCCGTGCAGGCAGAGGACCTGGCAGATTATTTCTGCCAGCAGCACTAC
GATACCCCCTATACATTTGGCGGCGGCACCAAGCTGGAGATCAAG
Amino acid sequence of 19F3H1L1 V_{L}: (SEQ ID NO: 8) 
DIVMTQSPSSLAMSVGERVTMSCKSSQSLLNSSNQKNYLAWYQQKPGQAPKLLVYFASTRE
SGVPDRFSGSGSGTDFTLTISSVQAEDLADYFCQQHYDTPYTFGGGTKLEIK
Nucleic acid sequences of 19F3H2L2 and 19F3H2L3 V_{H}: (SEQ ID NO: 9) 
CAGGTGCAGCTGGTGCAGTCTGGAGCAGAGGTGGTGAAGCCAGGAGCCTCTGTGAAGG
TGAGCTGTAAGGCCAGCGGCTACTCCTTCACCGGCTATACAATGAACTGGGTGAGGCAG
GCACCAGGACAGAATCTGGAGTGGATCGGCCTGATCAACCCTTACAATGCCGGCACCTCT
TATAACCAGAAGTTTCAGGGCAAGGTGACCCTGACAGTGGACAAGTCCACCTCTACAGC
CTACATGGAGCTGAGCTCCCTGCGGAGCGAGGATACAGCCGTGTACTATTGCGCCCGGTC
CGAGTACAGATATGGCGGCGACTACTTTGATTATTGGGGCCAGGGCACCACACTGACCGT
GTCTAGC
Amino acid sequence of 19F3H2L2 and 19F3H2L3 V_{H}: (SEQ ID NO: 10) 
QVQLVQSGAEVVKPGASVKVSCKASGYSFTGYTMNWVRQAPGQNLEWIGLINPYNAGTSY
NQKFQGKVTLTVDKSTSTAYMELSSLRSEDTAVYYCARSEYRYGGDYFDYWGQGTTLTVSS
Nucleic acid sequence of 19F3H2L2 V_{L}: (SEQ ID NO: 11) 
GACATCGTGATGACCCAGTCCCCAAGCTCCCTGGCCGTGTCTGTGGGAGAGCGGGTGAC
AATCTCCTGTAAGTCTAGCCAGTCTCTGCTGAACTCCTCTAATCAGAAGAACTACCTGGC
CTGGTATCAGCAGAAGCCCGGCCAGGCCCCTAAGCTGCTGATCTACTTCGCCTCTACCA
GGGAGAGCGGAGTGCCAGACAGATTCTCTGGCAGCGGCTCCGGCACAGACTTCACCCT
GACAATCAGCTCCGTGCAGGCAGAGGACGTGGCAGATTACTATTGCCAGCAGCACTAC
GATACCCCCTATACATTTGGCGGCGGCACCAAGCTGGAGATCAAG
Amino acid sequence of 19F3H2L2 V_{L}: (SEQ ID NO: 12) 
DIVMTQSPSSLAVSVGERVTISCKSSQSLLNSSNQKNYLAWYQQKPGQAPKLLIYFASTRES
GVPDRFSGSGSGTDFTLTISSVQAEDVADYYCQQHYDTPYTFGGGTKLEIK
Nucleic acid sequence of 19F3H2L3 V_{L}: (SEQ ID NO: 13) 
GACATCGTGATGACCCAGTCCCCAAGCTCCCTGGCCGTGTCTGTGGGAGAGCGGGTGAC
AATCTCCTGTAAGTCTAGCCAGTCTCTGCTGAACTCCTCTAATCAGAAGAACTACCTGGC
CTGGTATCAGCAGAAGCCCGGCCAGGCCCCTAAGCTGCTGATCTACTTCGCCTCTACCAG
GGAGAGCGGAGTGCCAGACAGATTCTCTGGCAGCGGCTCCGGCACAGACTTCACCCTGA
CAATCAGCTCCCTGCAGGCAGAGGACGTGGCCGTGTACTATTGCCAGCAGCACTACGATA
CCCCCTATACATTTGGCGGCGGCACCAAGCTGGAGATCAAG
Amino acid sequence of 19F3H2L3 V_{L}: (SEQ ID NO: 14) 
DIVMTQSPSSLAVSVGERVTISCKSSQSLLNSSNQKNYLAWYQQKPGQAPKLLIYFASTRESG
VPDRFSGSGSGTDFTLTISSLQAEDVAVYYCQQHYDTPYTFGGGTKLEIK
CDRs of 19F3 and 19F3H2L3
HCDR1: GYSFTGYT (SEQ ID NO: 15)
HCDR2: INPYNAGT (SEQ ID NO: 16)
HCDR3: ARSEYRYGGDYFDY (SEQ ID NO: 17)
LCDR1: QSLLNSSNQKNY (SEQ ID NO: 18)
LCDR2: FAS (SEQ ID NO: 19)
LCDR3: QQHYDTPYT (SEQ ID NO: 20).
Amino acid sequence of CPI-006 heavy chain SEQ ID NO: 21 
QVQLVQSGAEVEKPGASVKVSCKASGYTFTSYWITWVRQAPGQGLEWMGDIYPGSGNTNY
NEKFKTRVTITADKSTSTAYMELSSLRSEDTAVYYCAKEGGLTTEDYALDYWGQGTLVTVSS
 
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYQSTYRVVS
VLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVM
HEALHNHYTQKSLSLSPGK
Amino acid sequence of CPI-006 light chain SEQ ID NO: 22 
EIVLTQSPATLSLSPGERATLSCRASKNVSTSGYSYMHWYQQKPGQAPRLLIYLASNLESGIPP
RFSGSGYGTDFTLTINNIESEDAAYYFCQHSRELPFTFGQGTKVEIKRTVAAPSVFIFPPSDEQL
KSGTASVVCLLNNFYPREAKVQWKVDNALQSGNSQESVTEQDSKDSTYSLSSTLTLSKADY
EKHKVYACEVTHQGLSSPVTKSFNRGEC
Full-length amino acid sequence of 19F3H2L3(hG1DM) heavy chain SEQ ID NO: 23 
QVQLVQSGAEVVKPGASVKVSCKASGYSFTGYTMNWVRQAPGQNLEWIGLINPYNAG
TSYNQKFQGKVTLTVDKSTSTAYMELSSLRSEDTAVYYCARSEYRYGGDYFDYWGQGTTLT
VSSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPEAAGGPS
VFLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKNQ
VSLTCLVKGFYPSDIAVEWESNGQPENNYKTTPPVLDSDGSFFLYSKLTVDKSRWQQGNVFS
CSVMHEALHNHYTQKSLSLSPGK
Full-length amino acid sequence of 19F3H2L3(hG1DM) light chain SEQ ID NO: 24 
DIVMTQSPSSLAVSVGERVTISCKSSQSLLNSSNQKNYLAWYQQKPGQAPKLLIYFASTR
ESGVPDRFSGSGSGTDFTLTISSLQAEDVAVYYCQQHYDTPYTFGGGTKLEIKRTVAAPSVFIF
PPSDEQLKSGTASVVCLLNNFYPREAKVQWKVDNALQSGNSQESVTEQDSKDSTYSLSSTLT
LSKADYEKHKVYACEVTHQGLSSPVTKSFNRGEC
Note: Underscored parts represent CDR sequences

## Claims

1. An anti-CD73 (e.g., human CD73) antibody or antigen-binding fragment thereof for treating diseases caused by coronavirus infection, wherein the anti-CD73 antibody contains:
HCDR1, HCDR2 and HCDR3 contained in the V_{H} shown in SEQ ID NO: 2, and LCDR1, LCDR2 and LCDR3 contained in the V_{L} shown in SEQ ID NO: 4,
preferably, the anti-CD73 antibody contains:
HCDR1, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 15, or an amino acid sequence having one or a plurality of (preferably 1, 2, or 3) conservative amino acid mutations (preferably substitutions, insertions, or deletions) as compared to the sequence, or consists thereof;
HCDR2, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 16, or an amino acid sequence having one or a plurality of (preferably 1, 2, or 3) conservative amino acid mutations (preferably substitutions, insertions, or deletions) as compared to the sequence, or consists thereof;
HCDR3, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 17, or an amino acid sequence having one or a plurality of (preferably 1, 2, or 3) conservative amino acid mutations (preferably substitutions, insertions, or deletions) as compared to the sequence, or consists thereof;
LCDR1, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 18, or an amino acid sequence having one or a plurality of (preferably 1, 2, or 3) conservative amino acid mutations (preferably substitutions, insertions, or deletions) as compared to the sequence, or consists thereof;
LCDR2, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 19, or an amino acid sequence having one or a plurality of (preferably 1, 2, or 3) conservative amino acid mutations (preferably substitutions, insertions, or deletions) as compared to the sequence, or consists thereof; and
LCDR3, containing an amino acid sequence, a sequence having at least 80%, 81%, 82%, 83%, 84%, 85%, 86%, 87%, 88%, 89%, 90%, preferably at least 91%, 92%, 93%, 94%, 95%, 96%, 97%, 98%, or 99% sequence identity thereto, as shown in SEQ ID NO: 20, or an amino acid sequence having one or a plurality of (preferably 1, 2, or 3) conservative amino acid mutations (preferably substitutions, insertions, or deletions) as compared to the sequence, or consists thereof.

2. The anti-CD73 antibody or antigen-binding fragment thereof according to Claim 1, the V_{H} of the antibody contains the following sequence, or consists thereof:
SEQ ID NO: 2, SEQ ID NO: 6, or SEQ ID NO: 10, a sequence having at least 80%, 81%, 82%, 83%, 84%, 85%, 86%, 87%, 88%, 89%, or 90%, preferably at least 91%, 92%, 93%, 94%, 95%, 96%, 97%, 98%, or 99% sequence identity thereto, or an amino acid sequence having one or a plurality of (preferably 1, 2, 3, 4, 5, 6, 7, 8, 9, or 10) conservative amino acid mutations (preferably substitutions, insertions, or deletions) as compared to the amino acid sequence as shown in SEQ ID NO: 2, SEQ ID NO: 6 or SEQ ID NO: 10; and
the V_{L} of the antibody contains the following sequence, or consists thereof:
SEQ ID NO: 4, SEQ ID NO: 8, SEQ ID NO: 12 or SEQ ID NO: 14, a sequence having at least 80%, 81%, 82%, 83%, 84%, 85%, 86%, 87%, 88%, 89%, or 90%, preferably at least 91%, 92%, 93%, 94%, 95%, 96%, 97%, 98%, or 99% sequence identity thereto, or an amino acid sequence having one or a plurality of (preferably 1, 2, 3, 4, 5, 6, 7, 8, 9, or 10) conservative amino acid mutations (preferably substitutions, insertions, or deletions) as compared to the amino acid sequence as shown in SEQ ID NO: 4, SEQ ID NO: 8, SEQ ID NO: 12 or SEQ ID NO: 14.

3. The anti-CD73 antibody or antigen binding fragment thereof according to Claim 1 or 2, wherein
the amino acid sequence of the heavy chain variable region of the antibody is as shown in SEQ ID NO: 2, and the amino acid sequence of the light chain variable region of the antibody is as shown in SEQ ID NO: 4;
the amino acid sequence of the heavy chain variable region of the antibody is as shown in SEQ ID NO: 6, and the amino acid sequence of the light chain variable region of the antibody is as shown in SEQ ID NO: 8;
the amino acid sequence of the heavy chain variable region of the antibody is as shown in SEQ ID NO: 10, and the amino acid sequence of the light chain variable region of the antibody is as shown in SEQ ID NO: 12; or
the amino acid sequence of the heavy chain variable region of the antibody is as shown in SEQ ID NO: 10, and the amino acid sequence of the light chain variable region of the antibody is as shown in SEQ ID NO: 14.

4. The anti-CD73 antibody or antigen-binding fragment thereof according to Claim 1 or 2, wherein the C_{H} of the antibody is the Ig gamma-1 chain C region, ACCESSION: P01857; the C_{L} is the Ig kappa chain C region, ACCESSION: P01834.

5. The anti-CD73 antibody or antigen-binding fragment thereof according to Claim 1 or 2, wherein the antibody is a monoclonal antibody (preferably, the anti-CD73 antibody is a monoclonal antibody secreted by a hybridoma of preservation number CCTCC NO: C2018137), a humanized antibody, a chimeric antibody, or a multispecific antibody (e.g., a bispecific antibody), preferably, the heavy chain amino acid sequence of the antibody is as shown as SEQ ID NO: 23 and the light chain amino acid sequence is as shown as SEQ ID NO: 24.

6. The anti-CD73 antibody or antigen-binding fragment thereof according to Claim 1 or 2, wherein the antigen-binding fragment is selected from a Fab, a Fab', a F(ab')₂, a Fd, a Fv, a dAb, a Fab/c, a CDR fragment, a single chain antibody (e.g., scFv), a humanized antibody, a chimeric antibody, or a bispecific antibody.

7. A conjugate, for treating diseases caused by coronavirus infection, containing the anti-CD73 antibody or antigen-binding fragment thereof and a coupling portion according to any of Claims 1 - 6, in which the coupling portion is a purification tag (such as a His tag), a detectable label or a small molecule drug; preferably, the coupling portion is a radionuclide, a fluorescent substance, a chemiluminescent substance, a colored substance, polyethylene glycol or an enzyme; preferably, the small molecule drug is a small molecule cytotoxic drug; more preferably, the small molecule drug is a tumor chemotherapy drug, more preferably, the anti-CD73 antibody or antigen-binding fragment thereof is linked to the small molecule drug by a linker; for example, the linker is a hydrazone bond, a disulfide bond, or a peptide bond; more preferably, the anti-CD73 antibody or antigen-binding fragment thereof is linked to the small molecule drug at a certain molar ratio; for example, the molar ratio is 1: (2 - 4).

8. A fusion protein or a multispecific antibody (preferably a bispecific antibody) for treating diseases caused by coronavirus infection, containing the anti-CD73 antibody or antigen-binding fragment thereof according to any of Claims 1-6.

9. A kit, comprising the anti-CD73 antibody or antigen-binding fragment thereof according to any of Claims 1-6, the conjugate according to Claim 7, or the fusion protein or multispecific antibody according to Claim 8; preferably, the kit further comprises a secondary antibody or an antigen used as a vaccine, the secondary antibody specifically recognizing the anti-CD73 antibody or is for treating a virus (preferably a Coronaviridae viral infection), the antigen used as a vaccine is selected from virus, bacteria, fungi, rickettsia, chlamydia, mycoplasma, parasite, prion or tumor; optionally, the secondary antibody further comprises a detectable label, for example, a radionuclide, a fluorescent substance, a chemiluminescent substance, a colored substance, or enzyme; preferably, the kit is used to detect the presence of CD73 in a sample or the level thereof.

10. A pharmaceutical composition or single drug dosage unit for treating diseases caused by coronavirus infection, containing the anti-CD73 antibody or antigen-binding fragment thereof according to any of Claims 1 - 6, the conjugate according to Claim 7, or the fusion protein or multispecific antibody according to Claim 8; optionally, the pharmaceutical composition or single drug dosage unit further comprises a pharmaceutically acceptable vector and/or excipient. Preferably, the pharmaceutical composition is in a form suitable for administration by subcutaneous injection, intradermal injection, intravenous injection, intramuscular injection or intralesional injection.

11. The purpose of use of the anti-CD73 antibody or antigen-binding fragment thereof according to Claims 1 - 6, the conjugate, according to Claim 7, or the fusion protein or multispecific antibody according to Claim 8 in the preparation of a drug or kit for treating and/or preventing coronavirus infection, which is preferably selected from novel coronaviruses SARS-CoV-2, HCoV-229E, HCoV-OC43, HCoV-NL63, HCoV-HKU1, SARS-CoV, and/or MERS-CoV.

12. A method for treating and/or preventing coronavirus infection, comprising administering an effective dose of the anti-CD73 antibody or antigen-binding fragment thereof according to any of Claims 1 - 6, the conjugate according to Claim 7, or the fusion protein or multispecific antibody according to Claim 8 to a subject or patient, preferably, the Coronaviridae virus is selected from novel coronaviruses SARS-CoV-2, HCoV-229E, HCoV-OC43, HCoV-NL63, HCoV-HKU1, SARS-CoV, and/or MERS-CoV; more preferably, an antiviral drug is concurrently or sequentially administered to a subject or patient, preferably, the antiviral drug is an RNA virus inhibitor.

13. The method according to Claim 12, more preferably, the effective dose of the anti-CD73 antibody or antigen-binding fragment thereof, the conjugate, fusion protein, or multispecific antibody is 0.001 mg - 1,000 mg, more preferably 0.001 mg - 900 mg, 0.001 mg - 800 mg, 0.001 mg - 700 mg, 0.001 mg - 600 mg, 0.001 mg - 500 mg, 0.001 mg - 400 mg, 0.001 mg - 300 mg, 0.001 mg - 200 mg, or 0.001 mg - 100 mg, most preferably 100 mg, 200 mg, 300 mg, 400 mg, 500 mg, 600 mg, 700 mg, 800 mg, 900 mg or 1,000 mg, or the effective dose is 0.1 - 100 mg/kg, preferably 1 - 90 mg/kg, 1 - 80 mg/kg, 1-70 mg/kg, 1 - 60 mg/kg, 1-50 mg/kg, 1 - 40 mg/kg, 1-30 mg/kg, 1 - 20 mg/kg, or 1 - 10 mg/kg based on the weight of the subject or patient.

14. The method according to Claim 12, the effective dose of the antiviral drug is 100 - 2,400 mg, preferably 100 mg - 2,300 mg, 100 mg - 2,200 mg, 100 mg - 2,100 mg, 100 mg - 2,000 mg, 100 mg - 1,900 mg, 100 mg - 1,800 mg, 100 mg - 1,700 mg, 100 mg - 1,600 mg, 100 mg - 1800 mg, 100 mg - 1,800 mg, 100 mg - 1,800 mg, 100 mg - 1,800 mg, 100 mg - 1,800 mg, more preferably 100 mg, 200 mg, 300 mg, 400 mg, 500 mg, 600 mg, 700 mg, 800 mg, 900 mg, or 1,000 mg; alternatively, the effective dose of the antiviral drug is 0.1 - 100 mg/kg, preferably 1 - 90 mg/kg, 1-80 mg/kg, 1-70 mg/kg, 1-60 mg/kg, 1-50 mg/kg, 1 - 40 mg/kg, 1-30 mg/kg, 1-20 mg/kg, or 1 - 10 mg/kg, based on the weight of the subject or patient.

15. The anti-CD73 antibody or antigen-binding fragment thereof according to any of Claims 1 - 6, the conjugate according to Claim 7, or the fusion protein or multispecific antibody according to Claim 8, for use as an auxiliary to enhance the immune response of an organism to an antigen.

16. The anti-CD73 antibody or antigen-binding fragment thereof according to any of Claims 1 - 6, the conjugate according to Claim 7, or the fusion protein or multispecific antibody according to Claim 8, wherein the antigen is derived from virus, bacteria, fungi, rickettsia, chlamydia, mycoplasma, parasite, prion, or tumor;
preferably, the virus includes an RNA virus and a DNA virus;
preferably, the RNA virus includes a Coronaviridae virus;
preferably, the Coronaviridae virus includes 2019 novel coronavirus (2019-nCoV or SARS-CoV-2, which induces novel coronavirus pneumonia COVID-19), HCoV-229E, HCoV-OC43, HCoV-NL63, HCoV-HKU1, SARS-CoV (which induces severe acute respiratory syndrome), and MERS-CoV (which induces Middle East respiratory syndrome).

17. A method of increasing vaccine efficacy or enhancing an organism's responsiveness to a vaccine, comprising administering the anti-CD73 antibody or antigen-binding fragment according to any of Claims 1 - 6, the conjugate according to Claim 7, the fusion protein or multispecific antibody according to Claim 8 to the subject during, before, or after vaccination, preferably the antigen contained in the vaccine is derived from a virus, bacteria, fungi, rickettsia, chlamydia, mycoplasma, parasite, prion or tumor;
preferably, the virus includes an RNA virus and a DNA virus;
preferably, the RNA virus includes a Coronaviridae virus;
preferably, the Coronaviridae virus includes 2019 novel coronavirus (2019-nCoV or SARS-CoV-2, which induces novel coronavirus pneumonia COVID-19), HCoV-229E, HCoV-OC43, HCoV-NL63, HCoV-HKU1, SARS-CoV (which induces severe acute respiratory syndrome), and MERS-CoV (which induces Middle East respiratory syndrome).

18. The method according to Claim 17, wherein the anti-CD73 antibody or antigen-binding fragment thereof according to any Claims 1 - 6, the conjugate according to Claim 7, or the fusion protein or multispecific antibody according to Claim 8 is administered (preferably intravenously) one or more times.
